# EUROPEAN PATENT SPECIFICATION

(11) **EP 3 454 842 B1**
(45) Date of publication and mention of the grant of the patent: **08.07.2020**
(21) Application number: 17726564.2
(22) Date of filing: 12.05.2017
(51) Int. Cl.: A61K 31/00, G01N 33/564

(54) **USE OF ANTI-CD26 ANTIBODY LEVELS AS AUTOIMMUNE AND/OR INFLAMMATORY DISEASE BIOMARKERS**
VERWENDUNG VON ANTI-CD26-ANTIKÖRPERKONZENTRATIONEN ALS BIOMARKER FÜR AUTOIMMUN- UND/ODER ENTZÜNDUNGSKRANKHEITEN
UTILISATION DE TAUX D'ANTICORPS ANTI-CD26 EN TANT QUE BIOMARQUEURS DE MALADIES AUTO-IMMUNES ET/OU INFLAMMATOIRES

(30) Priority: 12.05.2016 ES 201630620
(43) Date of publication of application: 20.03.2019
(73) Proprietor: Servizo Galego De Saúde (Sergas), 15703 Santiago de Compostela , A Coruña (ES); Universidade de Santiago de Compostela, 15782 Santiago de Compostela (ES)
(72) Inventor: PEGO REIGOSA, José María, 15703 Santiago de Compostela- A Coruña (ES); CORDERO SANTAMARÍA, Óscar, 15782 Santiago de Compostela (ES); VARELA CALVIÑO, Rubén, 15782 Santiago de Compostela (ES)
(74) Representative: Hoffmann Eitle
(86) International application number: PCT/EP2017/061537
(87) International publication number: WO 2017/194779

(56) References cited:
- US-A1- 2006 177 886
- US-A1- 2014 194 308
- JASVINDER A. SINGH ET AL: "2015 American College of Rheumatology Guideline for the Treatment of Rheumatoid Arthritis : ACR RA TREATMENT RECOMMENDATIONS", ARTHRITIS & RHEUMATOLOGY (HOBOKEN), vol. 68, no. 1, 6 November 2015 (2015-11-06), pages 1-26, XP055391500, US ISSN: 2326-5191, DOI: 10.1002/art.39480
- M Cuchacovich ET AL: "Characterization of human serum dipeptidyl peptidase IV (CD26) and analysis of its autoantibodies in patients with rheumatoid arthritis and other autoimmune diseases", Clinical and experimental rheumatology, 1 November 2001 (2001-11-01), page 673, XP055391383, Italy Retrieved from the Internet: URL:http://www.clinexprheumatol.org/articl e.asp?a=1196
- VERHEUL M K ET AL: "Biomarkers for rheumatoid and psoriatic arthritis", CLINICAL IMMUNOLOGY, vol. 161, no. 1, 28 April 2015 (2015-04-28), pages 2-10, XP029321503, ISSN: 1521-6616, DOI: 10.1016/J.CLIM.2015.04.005
- KLAARTJE SOMERS ET AL: "Novel autoantibody markers for early and seronegative rheumatoid arthritis", JOURNAL OF AUTOIMMUNITY, LONDON, GB, vol. 36, no. 1, 10 October 2010 (2010-10-10), pages 33-46, XP028130655, ISSN: 0896-8411, DOI: 10.1016/J.JAUT.2010.10.003 [retrieved on 2010-10-18]
- OSCAR J. CORDERO ET AL: "CD26 Expression on T Helper Populations and sCD26 Serum Levels in Patients with Rheumatoid Arthritis", PLOS ONE, vol. 10, no. 7, 15 July 2015 (2015-07-15), page e0131992, XP055391284, DOI: 10.1371/journal.pone.0131992
- CONIGLIARO P ET AL: "Autoantibodies in inflammatory arthritis", AUTOIMMUNITY REVIEWS, ELSEVIER, AMSTERDAM, NL, vol. 15, no. 7, 9 March 2016 (2016-03-09), pages 673-683, XP029541466, ISSN: 1568-9972, DOI: 10.1016/J.AUTREV.2016.03.003
- OSCAR J. CORDERO ET AL: "Anti-CD26 autoantibodies are involved in rheumatoid arthritis and show potential clinical interest", CLINICAL BIOCHEMISTRY, 1 June 2017 (2017-06-01), XP055391421, US, CA ISSN: 0009-9120, DOI: 10.1016/j.clinbiochem.2017.06.001

### SEQUENCE LISTING

<110> Servizo Galego de Saúde (SERGAS) Universidade de Santiago de Compostela (USC)
<120> USE OF ANTI-CD26 ANTIBODY LEVELS AS AUTOIMMUNE AND/OR INFLAMMATORY DISEASE BIOMARKERS
<130> 902 170
<160> 2
<170> PatentIn version 3.5
<210> 1
   <211> 766
   <212> PRT
   <213> Homo sapiens
<220>
   <221> Dipeptidyl peptidase 4
   <222> (1)..(766)
   <223> membrane form
<400> 1
   Met Lys Thr Pro Trp Lys Val Leu Leu Gly Leu Leu Gly Ala Ala Ala
   1               5                   10                  15
   Leu Val Thr Ile Ile Thr Val Pro Val Val Leu Leu Asn Lys Gly Thr
               20                  25                  30
   Asp Asp Ala Thr Ala Asp Ser Arg Lys Thr Tyr Thr Leu Thr Asp Tyr
           35                  40                  45
   Leu Lys Asn Thr Tyr Arg Leu Lys Leu Tyr Ser Leu Arg Trp Ile Ser
       50                  55                  60
   Asp His Glu Tyr Leu Tyr Lys Gln Glu Asn Asn Ile Leu Val Phe Asn
   65                  70                  75                  80
   Ala Glu Tyr Gly Asn Ser Ser Val Phe Leu Glu Asn Ser Thr Phe Asp
                   85                  90                  95
   Glu Phe Gly His Ser Ile Asn Asp Tyr Ser Ile Ser Pro Asp Gly Gln
               100                 105                 110
   Phe Ile Leu Leu Glu Tyr Asn Tyr Val Lys Gln Trp Arg His Ser Tyr
           115                 120                 125
   Thr Ala Ser Tyr Asp Ile Tyr Asp Leu Asn Lys Arg Gln Leu Ile Thr
       130                 135                 140
   Glu Glu Arg Ile Pro Asn Asn Thr Gln Trp Val Thr Trp Ser Pro Val
   145                 150                 155                 160
   Gly His Lys Leu Ala Tyr Val Trp Asn Asn Asp Ile Tyr Val Lys Ile
                   165                 170                 175
   Glu Pro Asn Leu Pro Ser Tyr Arg Ile Thr Trp Thr Gly Lys Glu Asp
               180                 185                 190
   Ile Ile Tyr Asn Gly Ile Thr Asp Trp Val Tyr Glu Glu Glu Val Phe
           195                 200                 205
   Ser Ala Tyr Ser Ala Leu Trp Trp Ser Pro Asn Gly Thr Phe Leu Ala
       210                 215                 220
   Tyr Ala Gln Phe Asn Asp Thr Glu Val Pro Leu Ile Glu Tyr Ser Phe
   225                 230                 235                 240
   Tyr Ser Asp Glu Ser Leu Gln Tyr Pro Lys Thr Val Arg Val Pro Tyr
                   245                 250                 255
   Pro Lys Ala Gly Ala Val Asn Pro Thr Val Lys Phe Phe Val Val Asn
               260                 265                 270
   Thr Asp Ser Leu Ser Ser Val Thr Asn Ala Thr Ser Ile Gln Ile Thr
           275                 280                 285
   Ala Pro Ala Ser Met Leu Ile Gly Asp His Tyr Leu Cys Asp Val Thr
       290                 295                 300
   Trp Ala Thr Gln Glu Arg Ile Ser Leu Gln Trp Leu Arg Arg Ile Gln
   305                 310                 315                 320
   Asn Tyr Ser Val Met Asp Ile Cys Asp Tyr Asp Glu Ser Ser Gly Arg
                   325                 330                 335
   Trp Asn Cys Leu Val Ala Arg Gln His Ile Glu Met Ser Thr Thr Gly
               340                 345                 350
   Trp Val Gly Arg Phe Arg Pro Ser Glu Pro His Phe Thr Leu Asp Gly
           355                 360                 365
   Asn Ser Phe Tyr Lys Ile Ile Ser Asn Glu Glu Gly Tyr Arg His Ile
       370                 375                 380
   Cys Tyr Phe Gln Ile Asp Lys Lys Asp Cys Thr Phe Ile Thr Lys Gly
   385                 390                 395                 400
   Thr Trp Glu Val Ile Gly Ile Glu Ala Leu Thr Ser Asp Tyr Leu Tyr
   405 410 415
   Tyr Ile Ser Asn Glu Tyr Lys Gly Met Pro Gly Gly Arg Asn Leu Tyr
   420 425 430
   Lys Ile Gln Leu Ser Asp Tyr Thr Lys Val Thr Cys Leu Ser Cys Glu
   435 440 445
   Leu Asn Pro Glu Arg Cys Gln Tyr Tyr Ser Val Ser Phe Ser Lys Glu
   450 455 460
   Ala Lys Tyr Tyr Gln Leu Arg Cys Ser Gly Pro Gly Leu Pro Leu Tyr
   465 470 475 480
   Thr Leu His Ser Ser Val Asn Asp Lys Gly Leu Arg Val Leu Glu Asp
   485 490 495
   Asn Ser Ala Leu Asp Lys Met Leu Gln Asn Val Gln Met Pro Ser Lys
   500 505 510
   Lys Leu Asp Phe Ile Ile Leu Asn Glu Thr Lys Phe Trp Tyr Gln Met
   515 520 525
   Ile Leu Pro Pro His Phe Asp Lys Ser Lys Lys Tyr Pro Leu Leu Leu
   530 535 540
   Asp Val Tyr Ala Gly Pro Cys Ser Gln Lys Ala Asp Thr Val Phe Arg
   545 550 555 560
   Leu Asn Trp Ala Thr Tyr Leu Ala Ser Thr Glu Asn Ile Ile Val Ala
   565 570 575
   Ser Phe Asp Gly Arg Gly Ser Gly Tyr Gln Gly Asp Lys Ile Met His
   580 585 590
   Ala Ile Asn Arg Arg Leu Gly Thr Phe Glu Val Glu Asp Gln Ile Glu
   595 600 605
   Ala Ala Arg Gln Phe Ser Lys Met Gly Phe Val Asp Asn Lys Arg Ile
   610 615 620
   Ala Ile Trp Gly Trp Ser Tyr Gly Gly Tyr Val Thr Ser Met Val Leu
   625 630 635 640
   Gly Ser Gly Ser Gly Val Phe Lys Cys Gly Ile Ala Val Ala Pro Val
   645 650 655
   Ser Arg Trp Glu Tyr Tyr Asp Ser Val Tyr Thr Glu Arg Tyr Met Gly
               660                 665                 670
   Leu Pro Thr Pro Glu Asp Asn Leu Asp His Tyr Arg Asn Ser Thr Val
           675                 680                 685
   Met Ser Arg Ala Glu Asn Phe Lys Gln Val Glu Tyr Leu Leu Ile His
       690                 695                 700
   Gly Thr Ala Asp Asp Asn Val His Phe Gln Gln Ser Ala Gln Ile Ser
   705                 710                 715                 720
   Lys Ala Leu Val Asp Val Gly Val Asp Phe Gln Ala Met Trp Tyr Thr
                   725                 730                 735
   Asp Glu Asp His Gly Ile Ala Ser Ser Thr Ala His Gln His Ile Tyr
               740                 745                 750
   Thr His Met Ser His Phe Ile Lys Gln Cys Phe Ser Leu Pro
           755                 760                 765
<210> 2
   <211> 727
   <212> PRT
   <213> Homo sapiens
<220>
   <221> Dipeptidyl peptidase 4
   <222> (1)..(727)
   <223> soluble form
<400> 2
   Arg Lys Thr Tyr Thr Leu Thr Asp Tyr Leu Lys Asn Thr Tyr Arg Leu
   1               5                   10                  15
   Lys Leu Tyr Ser Leu Arg Trp Ile Ser Asp His Glu Tyr Leu Tyr Lys
               20                  25                  30
   Gln Glu Asn Asn Ile Leu Val Phe Asn Ala Glu Tyr Gly Asn Ser Ser
           35                  40                  45
   Val Phe Leu Glu Asn Ser Thr Phe Asp Glu Phe Gly His Ser Ile Asn
       50                  55                  60
   Asp Tyr Ser Ile Ser Pro Asp Gly Gln Phe Ile Leu Leu Glu Tyr Asn
   65                  70                  75                  80
   Tyr Val Lys Gln Trp Arg His Ser Tyr Thr Ala Ser Tyr Asp Ile Tyr
   85 90 95
   Asp Leu Asn Lys Arg Gln Leu Ile Thr Glu Glu Arg Ile Pro Asn Asn
   100 105 110
   Thr Gln Trp Val Thr Trp Ser Pro Val Gly His Lys Leu Ala Tyr Val
   115 120 125
   Trp Asn Asn Asp Ile Tyr Val Lys Ile Glu Pro Asn Leu Pro Ser Tyr
   130 135 140
   Arg Ile Thr Trp Thr Gly Lys Glu Asp Ile Ile Tyr Asn Gly Ile Thr
   145 150 155 160
   Asp Trp Val Tyr Glu Glu Glu Val Phe Ser Ala Tyr Ser Ala Leu Trp
   165 170 175
   Trp Ser Pro Asn Gly Thr Phe Leu Ala Tyr Ala Gln Phe Asn Asp Thr
   180 185 190
   Glu Val Pro Leu Ile Glu Tyr Ser Phe Tyr Ser Asp Glu Ser Leu Gln
   195 200 205
   Tyr Pro Lys Thr Val Arg Val Pro Tyr Pro Lys Ala Gly Ala Val Asn
   210 215 220
   Pro Thr Val Lys Phe Phe Val Val Asn Thr Asp Ser Leu Ser Ser Val
   225 230 235 240
   Thr Asn Ala Thr Ser Ile Gln Ile Thr Ala Pro Ala Ser Met Leu Ile
   245 250 255
   Gly Asp His Tyr Leu Cys Asp Val Thr Trp Ala Thr Gln Glu Arg Ile
   260 265 270
   Ser Leu Gln Trp Leu Arg Arg Ile Gln Asn Tyr Ser Val Met Asp Ile
   275 280 285
   Cys Asp Tyr Asp Glu Ser Ser Gly Arg Trp Asn Cys Leu Val Ala Arg
   290 295 300
   Gln His Ile Glu Met Ser Thr Thr Gly Trp Val Gly Arg Phe Arg Pro
   305 310 315 320
   Ser Glu Pro His Phe Thr Leu Asp Gly Asn Ser Phe Tyr Lys Ile Ile
   325 330 335
   Ser Asn Glu Glu Gly Tyr Arg His Ile Cys Tyr Phe Gln Ile Asp Lys
               340                 345                 350
   Lys Asp Cys Thr Phe Ile Thr Lys Gly Thr Trp Glu Val Ile Gly Ile
           355                 360                 365
   Glu Ala Leu Thr Ser Asp Tyr Leu Tyr Tyr Ile Ser Asn Glu Tyr Lys
       370                 375                 380
   Gly Met Pro Gly Gly Arg Asn Leu Tyr Lys Ile Gln Leu Ser Asp Tyr
   385                 390                 395                 400
   Thr Lys Val Thr Cys Leu Ser Cys Glu Leu Asn Pro Glu Arg Cys Gln
                   405                 410                 415
   Tyr Tyr Ser Val Ser Phe Ser Lys Glu Ala Lys Tyr Tyr Gln Leu Arg
               420                 425                 430
   Cys Ser Gly Pro Gly Leu Pro Leu Tyr Thr Leu His Ser Ser Val Asn
           435                 440                 445
   Asp Lys Gly Leu Arg Val Leu Glu Asp Asn Ser Ala Leu Asp Lys Met
       450                 455                 460
   Leu Gln Asn Val Gln Met Pro Ser Lys Lys Leu Asp Phe Ile Ile Leu
   465                 470                 475                 480
   Asn Glu Thr Lys Phe Trp Tyr Gln Met Ile Leu Pro Pro His Phe Asp
                   485                 490                 495
   Lys Ser Lys Lys Tyr Pro Leu Leu Leu Asp Val Tyr Ala Gly Pro Cys
               500                 505                 510
   Ser Gln Lys Ala Asp Thr Val Phe Arg Leu Asn Trp Ala Thr Tyr Leu
           515                 520                 525
   Ala Ser Thr Glu Asn Ile Ile Val Ala Ser Phe Asp Gly Arg Gly Ser
       530                 535                 540
   Gly Tyr Gln Gly Asp Lys Ile Met His Ala Ile Asn Arg Arg Leu Gly
   545                 550                 555                 560
   Thr Phe Glu Val Glu Asp Gln Ile Glu Ala Ala Arg Gln Phe Ser Lys
                   565                 570                 575
   Met Gly Phe Val Asp Asn Lys Arg Ile Ala Ile Trp Gly Trp Ser Tyr
               580                 585                 590
   Gly Gly Tyr Val Thr Ser Met Val Leu Gly Ser Gly Ser Gly Val Phe
           595                 600                 605
   Lys Cys Gly Ile Ala Val Ala Pro Val Ser Arg Trp Glu Tyr Tyr Asp
       610                 615                 620
   Ser Val Tyr Thr Glu Arg Tyr Met Gly Leu Pro Thr Pro Glu Asp Asn
   625                 630                 635                 640
   Leu Asp His Tyr Arg Asn Ser Thr Val Met Ser Arg Ala Glu Asn Phe
                   645                 650                 655
   Lys Gln Val Glu Tyr Leu Leu Ile His Gly Thr Ala Asp Asp Asn Val
               660                 665                 670
   His Phe Gln Gln Ser Ala Gln Ile Ser Lys Ala Leu Val Asp Val Gly
           675                 680                 685
   Val Asp Phe Gln Ala Met Trp Tyr Thr Asp Glu Asp His Gly Ile Ala
       690                 695                 700
   Ser Ser Thr Ala His Gln His Ile Tyr Thr His Met Ser His Phe Ile
   705                 710                 715                 720
   Lys Gln Cys Phe Ser Leu Pro
                   725

## Description

### FIELD OF INVENTION

The present invention can be included in the field of personalized medicine, wherein specific biomarkers are used for the screening, diagnosis, prognosis, predicting treatment outcome, and/or monitoring of a given disease or disorder. Specifically, anti-CD26 antibodies are used in the present invention as markers for the screening, diagnosis or monitoring of human subjects having, or suspected to have an autoimmune and/or inflammatory disease, such as rheumatoid arthritis, in particular those in an early stage of the disease.

### BACKGROUND OF THE INVENTION

Rheumatoid arthritis (RA) is considered an autoimmune and inflammatory disease. It is the most common form of chronic inflammatory joint disease and affects 1-2% of the general population. Disease is characterized by joint swelling, joint tenderness, and destruction of synovial joints, leading to severe disability and premature mortality.

The optimal use of disease modifying antirheumatic drugs (DMARDs), such as methotrexate, and the incorporation into the RA therapeutic scheme of new biologic agents in the last ten years has dramatically enhanced the success of RA management. In this respect, there is a growing body of evidence indicating that joint destruction and functional decline (disability) in RA are improved by early, aggressive therapeutic intervention. Treating patients at a stage at which evolution of joint destruction can still be prevented would be ideal, thus the need for an unequivocal early diagnosis has become critical (Aletaha D et al. Arthritis Rheum. 2010. 62: 2569-2581).

The diagnosis of RA is still based on specific clinical parameters such as the number of affected joints or duration of symptoms. However, the early clinical presentation of RA is initially indistinguishable from other forms of arthritis (De Rooy DP et al. Rheumatology. 2011. 50:93-100).

Analytes or biomarkers such as erythrocyte sedimentation rate (ESR), C-reactive protein (CRP) concentration levels), rheumatoid factor auto-antibodies (EP0175270) and anti-mannose binding lectin (MBL) auto-antibodies (Gupta B et al. J Autoimmun. 2006. 27: 125-133), (Afzal Net al. Clin Lab. 2011. 57: 895-899, Takizawa Y et al. Ann Rheum Dis. 2006. 65: 1013-1020, Vossenaar ER et al. Arthritis Res Ther. 2004. 6: R142-R150) are been used with limited diagnostic value. Current tests for monitoring disease progression and responsiveness to therapy in RA have also sensitivity limitations (Gonzalez-Quintela A et al. Clinical and Experimental Immunology. 2007. 151: 42-50).

Antigens such as citrullinated vimentin, type II collagen, fibrinogen and alpha enolase have been reported to be associated with high titers of autoantibodies (ACPA) in the sera of patients with established disease (Ohmura K et al. Rheumatology. 2010. 49: 2298-2304, Vossenaar ER et al. Arthritis Res Ther. 2004. 6: 107-111). Anti-cyclic citrullinated peptides (or ACPA) are not only highly specific for RA, they also appear early in the disease process when diagnosis is most difficult and intervention most effective. However, there is a subset of the population negative for this biomarker (around 1/4). (Aletaha D et al. Arthritis Rheum. 2010. 62: 2569-2581). Sensitivity of these antibodies was about 80% in established RA compared with 55% in early RA and 40% in very early RA (Nicaise Roland Pet al., Arthritis Res Ther 2008;10(6):R142, Ohmura K et al. Rheumatology. 2010. 49: 2298-2304).

Recent investigations on ACPA are revealing information about the antigen specificity that initiates or perpetuates inflammatory autoimmune reactions before disease onset. For example, aberrant post-translational modifications of self-proteins have been reported to play a role in breaking T and B cell tolerance (Reynisdottir G et al. Arthritis Rheum. 2014. 66(1):31-9). Also, it has been described by some authors that ACPA immunity starts outside the joints, with lungs, lymph nodes and gingival tissue being identified as tissues where RA pathogenesis is initiated (Catrina Al et al. Nat Rev Rheumatol. 2014. 10(11):645-53; Demoruelle MK et al. Curr Opin Rheumatol. 2014. 26(1):64-71).

Levels of ACPA have been reported to be higher in synovial fluid compared to serum supporting a dominant synovial manifestation (Aletaha D et al. Arthritis Rheum. 2010. 62: 2569-2581, Snir O et al. Arthritis Rheum. 2010. 62: 44-52). In this context, researchers are now preferentially looking for self-antigens with high titers of auto-antibodies in serum in an attempt to understand RA pathogenesis and obtain tools for early diagnosis (Reynisdottir G et al. Arthritis Rheum. 2014. 66(1):31-9, Catrina Al et al. Nat Rev Rheumatol. 2014. 10(11):645-53, Snir O et al. Arthritis Rheum. 2010. 62: 44-52, Trouw LA et al. Autoimmun Rev. 2012. 12(2):318-22).

Human dipeptidyl peptidase IV (DPP-IV/CD26), a type II membrane glycoprotein with intrinsic DPP-IV activity, has been described as a key molecule in immune regulation and autoimmune diseases (Hosono et al., Modern Rheumatology 2003, 13(3), 199-204). DPP IV purified from human serum has been reported to lack the transmembrane domain of the membrane-bound enzyme; however, its enzymatic activity or adenosine deaminase (ADA) binding capacity is not impaired (Iwaki-Egawa S, et al., Jpn J Biochem 1998; 124: 428-33).

DPP-IV activity has been described as a contributing factor in the pathogenesis of rheumatoid arthritis (Busso N et al. Am J Pathol. 2005. 166: 433-42, Sedo A et al. Arthritis Res Ther. 2005. 7:253-69). DPP-IV as a result of its N-terminal X-Pro dipeptides cleaving activity can regulate chemotactic responses to inflammatory chemokines, including SDF-1, biologically active peptides such as NPY and VIP, recently implicated in RA, and is a neutrophil chemorepelent (Herlihy SE et al. Arthritis Rheumatol. 2015. 67(10):2634-8, Cordero OJ et al. PLoS One. 2015. 10(7):e0131992).

Several authors, including the inventors, (Cordero OJ et al. PLoS One. 2015. 10(7):e0131992, Cordero OJ et al. Rheumatol Int. 2001. 21: 69-74, Muscat C et al. Clin Exp Immunol. 1994. 98: 252-6, Ellingsen T et al. Scand J Immunol. 2007. 66: 451-7) have reported reduced levels of sCD26 and DPP-IV activity in RA patients and found important correlations between CD26 expression in some T cell subsets with disease activity (DAS28) and sCD26 serum levels (Cordero OJ et al. PLoS One. 2015. 10(7):e0131992).

Autoantibodies against human serum DPP-IV (sCD26) were predicted by Barr et al. (Barr VA et al. J Biol Chem. 1995. 270:27834-44) and were first described by Cuchacovich et al (Clin Exp Rheumatol. 2001. 19: 673-80). Cuchacovich et al determined the median serum levels of DPP-IV, the DPP-IV specific activity and the levels of anti-CD26 antibodies of the IgG, IgM and IgA isotypes in patients suffering from autoimmune and inflammatory diseases, namely RA, systemic lupus erythematosus (SLE) and primary Sjogren syndrome (SS). They found that DPP IV serum levels in RA patients were similar to those of normal controls; however, they were decreased in the sera of SLE and SS patients when compared with normal controls. Interestingly, while the concentration of DPP IV in the sera of RA patients appeared normal, its specific activity was significantly reduced. Furthermore, they investigated whether anti-DPP IV antibodies may modify enzyme activity. The results showed that sera levels of anti-DPP IV autoantibodies of the IgA class were significantly higher in the sera of RA, SLE and SS patients when compared with those of normal controls. Serum levels of anti-DPP IV autoantibodies of the IgG and IgM classes in the three groups of patients were however found not to be significantly different from those of normal controls. Cuchacovich et al concluded that only anti-DPP IV autoantibodies of the IgA class may have some relevance in the immune response against DPP IV.

US2006/0177886 relates to a method for diagnosing RA comprising measuring DPP-IV activity in a biological sample collected from a mammal. Notably, it reported a statistically significant increase (about 5-fold higher) of DPP-IV activity in RA patients with respect to osteoarthritis (OA) patients. There was not observed a correlation between the DPP-IV activity in RA and the severity of the disease.

Early detection and diagnosis of RA has been associated with a more positive outcome and is crucial for providing correct, evidence-based treatment, according to the stage of progression of the disease. From the clinician's perspective, early detection, accurate diagnosis and classification of the disease according to its stage of development would have potential benefits with respect to patient counselling, assessing disease prognosis, monitoring disease progression and relapses, and particularly with choosing the most appropriate treatment.

Despite significant advances having been made in the last years in the discovery of molecular and serological markers related to RA, there is an on-going need for improved methods for the early detection, accurate diagnosis, classification, study of the progression and/or prognosis of RA and related autoimmune and inflammatory diseases.

### SUMMARY OF THE INVENTION

IgA, IgM and IgG anti-CD26 titers were measured in a cohort of RA patients which were undergoing different biological and non-biological therapies (**Example 3**), and for which disease activity was previously assessed by the inventors as published in Cordero OJ et al. PLoS One. 2015. 10(7):e0131992. In the newly generated data, higher levels of anti-CD26 auto-antibody (ab) titers, around two-fold for each isotype, were found in said cohort with respect to healthy donors, whereas ratios with respective total Ig titers were different for each isotype. More specifically, IgA, IgM and IgG anti-CD26 titers and anti-CD26 / total antibodies IgG isotype ratio were found to be statistically significantly different compared to healthy donors (**Example 3.1**).

Based on this finding, the discriminating value of anti-CD26 antibodies between RA patients (undergoing different therapies) and healthy donors was determined in the whole cohort where the IgG titers showed a sensitivity of 82% and a specificity of 96% with chosen cut-offs (**Example 4.1**).

Moreover, the screening/diagnostic value was tested in the group undergoing DMARDs but no biological therapy (noBT), which would better resemble undiagnosed patients and/or patients closer to pre-RA in the sense that these therapies do not change CD26 expression. A sensitivity of 77% was found for the anti-CD26 IgG isotype and a sensitivity of 73% for the anti-CD26 IgM (**Example 4.2**). Only 2 out of 22 patients were negative for the three isotypes. These results point to the usefulness of these markers in the screening or diagnosis of RA.

In addition, correlations of anti-CD26 titers with disease activity parameters were found (**Example 6**), in particular the anti-CD26 antibodies of the three isotypes were found to correlate negatively with the Tender Joints Count (TCJ), suggesting a specific relation of these auto-antibodies with the joints. Also, the CD26 antigen used in the performed ELISA tests is not citrullinated suggesting that the detected auto-antibodies would recognize a sCD26 form which is not yet citrullinated. Taken together this data may suggest that anti-CD26 antibodies appear in an early stage of the RA pathogenesis.

Thus, in accordance with these particular findings, the present invention provides in a first aspect an *in vitro* method for the screening of a subject having, or suspected of having an inflammatory rheumatic disease comprising the following steps:
a) determining the levels of anti-CD26 IgG and/ or anti-CD26 IgM antibodies in an antibody-containing sample isolated from said subject;
b) comparing the levels in said antibody-containing sample with a reference value.

In a related aspect, the disclosure refers to an *in vitro* method for obtaining useful data for the diagnosis of an autoimmune and/or inflammatory disease in a subject, said method comprising the steps a) and b) of the first aspect.

In a further related aspect, the invention refers to an *in vitro* method for the diagnosis of an inflammatory rheumatic disease in a subject, said method comprising the steps a) and b) of the first aspect.

In an additional related aspect, the invention refers to an *in vitro* method for classifying subjects as i) healthy subjects or as ii) subjects having, or suspected of having an inflammatory rheumatic disease, said method comprising the steps a) and b) of the first aspect.

In another aspect, the invention relates to an *in vitro* method of disease monitoring and/or monitoring responsiveness to a treatment in a subject having or suspected to have an inflammatory rheumatic disease, said method comprising the steps a) and b) of the first aspect of the invention.

In a related aspect, the invention refers to an *in vitro* method for the classification of a subject as responder to a treatment to an inflammatory rheumatic disease, said method comprising the steps a) and b) of the first aspect of the invention.

In a further aspect, the present disclosure relates to a method of treating a subject having an autoimmune and/or inflammatory disease, said method comprising identifying the subject to be treated by a method comprising the steps a) and b) of the first aspect of the invention and
c) treating said subject suffering or suspected to suffer from an autoimmune and/or inflammatory disease.

In further aspect, the disclosure relates to an *in vitro* method for determining the stage of progression of an autoimmune and/or inflammatory disease in a subject, said method comprising the steps a) and b) of the first aspect of the invention.

In a related aspect, the disclosure refers to an *in vitro* method of determining a treatment for a subject suffering from an autoimmune and/or inflammatory disease according to its stage of progression comprising the steps a) and b) of the first aspect of the invention.

Use of *in vitro* determined anti-CD26 IgG and/ or anti-CD26 IgM and/or the anti-CD26 IgG antibody levels/total IgG antibody levels ratio, optionally in combination with *in vitro* determined anti-CD26 IgA antibody levels, in an antibody-containing sample isolated from a subject for the screening, diagnosis, and/or monitoring of an inflammatory rheumatic disease; and/or for monitoring the effectiveness of a treatment in an autoimmune and/or inflammatory disease.

In a further aspect, the disclosure relates to a kit comprising reagents for determining anti-CD26 antibody levels of the IgG isotype in an antibody-containing sample isolated from a subject, wherein said kit comprises:
a) a reagent for determining the levels of anti-CD26 antibodies of the IgG isotype;
b) optionally, instructions for the use of said reagent in determining the anti-CD26 antibody levels of the IgG isotype in an antibody-containing sample isolated from said subject.

In another aspect, the disclosure relates to a kit comprising reagents for determining anti-CD26 antibody levels of the IgM isotype in an antibody-containing sample isolated from a subject, wherein said kit comprises:
a) a reagent for determining the levels of anti-CD26 antibodies of the IgM isotype;
b) optionally, instructions for the use of said reagent in determining the anti-CD26 antibody levels of the IgM isotype in an antibody-containing sample isolated from said subject.

In still an additional aspect, the invention relates to the use of a kit of the preceding aspect for the screening, diagnosis, and/or monitoring of an inflammatory rheumatic disease; and/or for monitoring the effectiveness of a treatment in an antibody-containing sample isolated from a subject.

In a further aspect, the disclosure relates to an *in vitro* method for the screening, for obtaining useful data for the diagnosis, for the diagnosis or for the monitoring of a subject having, or suspected of having an autoimmune and/or inflammatory disease comprising the following steps:
a) determining the levels of total antibodies of the IgA isotype (total IgA antibody levels) in an antibody-containing sample isolated from said subject;
b) comparing the levels in said antibody-containing sample with a reference value.

In a further aspect, the present disclosure relates to a method of treating a subject having an autoimmune and/or inflammatory disease, said method comprising identifying the subject to be treated by a method comprising the steps of:
a) determining the levels of total antibodies of the IgA isotype (total IgA antibody levels) in an antibody-containing sample isolated from said subject;
b) comparing the levels in said antibody-containing sample with a reference value; and
c) treating said subject suffering or suspected to suffer from an autoimmune and/or inflammatory disease.

In an additional aspect, the disclosure relates to a kit comprising reagents for determining total antibody levels of the IgA isotype in an antibody-containing sample isolated from a subject, wherein said kit comprises:
a) a reagent for determining the levels of total antibody levels of the IgA isotype;
b) optionally, instructions for the use of said reagent in determining the total IgA antibody levels in an antibody-containing sample isolated from said subject.

In still an additional aspect, the invention relates to the use of a kit for the screening, diagnosis, and/or monitoring of an inflammatory rheumatic disease; and/or for monitoring the effectiveness of a treatment in an antibody-containing sample isolated from a subject, wherein said kit comprises reagents for determining total IgA antibody levels according to those kits described in the preceding aspect.

### BRIEF DESCRIPTION OF THE DRAWINGS

**Fig. 1****.** Cut off values for anti-CD26/DPP-IV auto-Ab titers and frequency of anomalous values in RA patients of the noBT group (patients under DMARD) and control donors. Titers of the IgM **(A),** IgG **(B)** and IgA **(C)** classes in both groups are shown. Cut-off values (20, 2.85 and 1.8 µg mL⁻¹, respectively).
**Fig. 2****.** Correlations between RA disease activity parameters and IgA anti-CD26/DPP-IV auto-Ab titers in the anti-TNF group. In **(A)** correlation of the IgA isotype with the TJC. In **(B)** correlation of the IgA isotype with the DAS28 score.
**Fig. 3****.** Correlations between RA disease activity parameters and IgG anti-CD26/DPP-IV auto-Ab titers in the anti-CD20 group. In **(A)** correlations of the IgG isotype with the TJC; in **(B)** with the SJC; in **(C)** with the DAS28 correlations of the IgA isotype with the DAS28 score, and in **(D)** with the PGA.

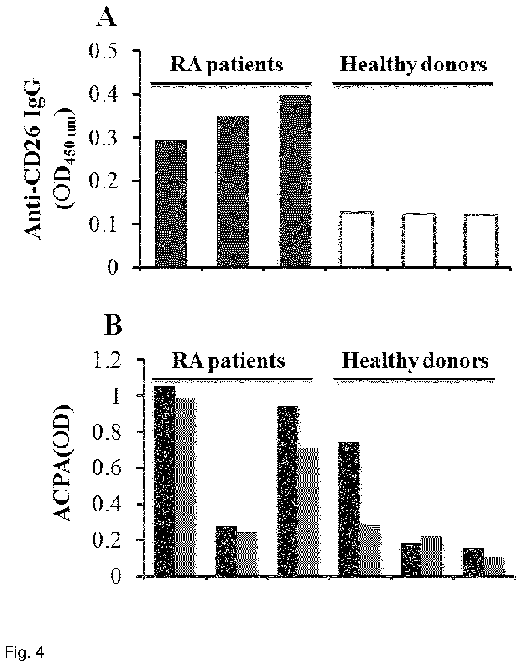

**Fig. 4****.** Anti-CD26/DPP-IV auto-Abs are not ACPA. In **(A)** supernatants recovered from the ACPA test after incubation of serum from 3 healthy donors and patients were then used for the anti-CD26 test. The same absorbance values were observed when serum samples were used directly in this anti-CD26 test (not shown). In **(B),** bars in dark gray show absorbance values of the ACPA test performed in serum for the same individuals (one control was also positive) and bars in clear gray show absorbance values of the ACPA test carried out in supernatants recovered from the anti-CD26 test of the same individuals; in one RA patient (3^{rd}) and in one healthy donor (1^{st}) differences were observed. Background values were extracted from the shown absorbance data.

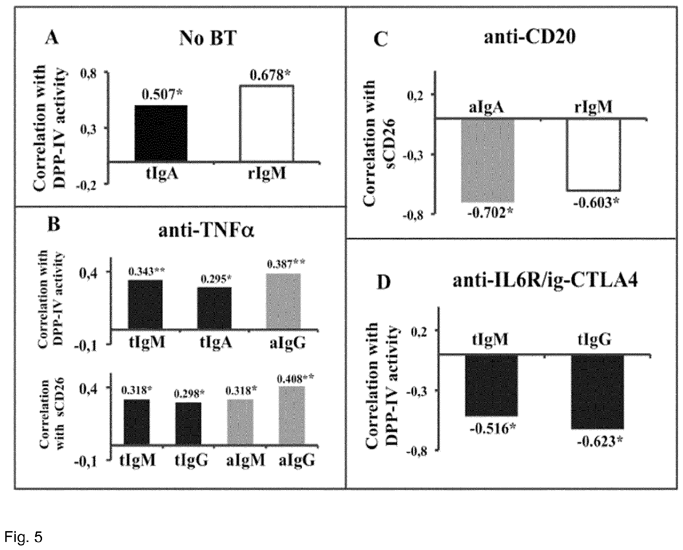

**Fig. 5****.** Correlations of anti-CD26/DPP-IV auto-Ab and total Ig titers of the three isotypes (A,G or M), or their ratios (auto-Ab/total Ig) with serum sCD26 concentration and DPP-IV enzymatic activities in the cohort of RA patients under different therapies. **(A)** Patients under DMARDs (no BT therapy), **(B), (C)** and **(D)** patients under biological therapy (BT): anti-TNFalpha, anti-CD20 and anti-IL6R/Ig-CTLA4 therapy, respectively. sCD26 refers to DPP-IV concentration. Numbers represent Pearson's coefficient, r, asterisks for statistical significance, *p<0.05, ** p<0.005). Only in the anti-TNF group there were some coincidences.
**Fig. 6****.** Effect of therapies on antibody titers are shown as ratios between Ig A, G or M isotypes. Columns 1-4 refer to samples of patients having received DMARDs; anti-TNF-a; anti-CD20; and anti-IL6R/Ig-CTLA4 respectively. In many cases, the effects on ratios of auto-Ab isotypes (lower row) were the opposite to that observed on ratios of total Ab isotypes (upper row), underlying the specificity of the anti-CD26 auto-Abs.
**Fig. 7****.** Receiver-operating-characteristic (ROC) curve for anti-CD26 IgM in the whole cohort. Area Under Curve (AUC) = 0.748. X axis represents 1 - Specificity. Y axis represents Sensitivity.
**Fig. 8****.** Receiver-operating-characteristic (ROC) curve for anti-CD26 IgG in the whole cohort. Area Under Curve (AUC) = 0.881. X axis represents 1 - Specificity. Y axis represents Sensitivity.
**Fig. 9****.** Receiver-operating-characteristic (ROC) curve for anti-CD26 IgA in the whole cohort. Area Under Curve (AUC) = 0.661. X axis represents 1 - Specificity. Y axis represents Sensitivity.
**Fig. 10****.** Receiver-operating-characteristic (ROC) curve for anti-CD26 IgG/total IgG in the whole cohort. Area Under Curve (AUC) = 0.741. X axis represents 1 - Specificity. Y axis represents Sensitivity.
**Fig. 11****.** Receiver-operating-characteristic (ROC) curve for anti-CD26 IgM in patients undergoing DMARDs but no biological therapy. Area Under Curve (AUC) = 0.751. X axis represents 1 - Specificity. Y axis represents Sensitivity.
**Fig. 12****.** Receiver-operating-characteristic (ROC) curve for anti-CD26 IgG in patients undergoing DMARDs but no biological therapy. Area Under Curve (AUC) = 0.846. X axis represents 1 - Specificity. Y axis represents Sensitivity.
**Fig. 13****.** Receiver-operating-characteristic (ROC) curve for anti-CD26 IgA in patients undergoing DMARDs but no biological therapy. Area Under Curve (AUC) = 0.672. X axis represents 1 - Specificity. Y axis represents Sensitivity.
**Fig. 14****.** Receiver-operating-characteristic (ROC) curve for anti-CD26 IgG/total IgG in patients undergoing DMARDs but no biological therapy. Area Under Curve (AUC) = 0.804. X axis represents 1 - Specificity. Y axis represents Sensitivity.
**Fig. 15****.** Receiver-operating-characteristic (ROC) curve for total IgA in the whole cohort. Area Under Curve (AUC) = 0.979. X axis represents 1 - Specificity. Y axis represents Sensitivity.
**Fig. 16****.** Receiver-operating-characteristic (ROC) curve for total IgA in in patients undergoing DMARDs but no biological therapy. Area Under Curve (AUC) = 0.947. X axis represents 1 - Specificity. Y axis represents Sensitivity.

### DETAILED DESCRIPTION OF THE INVENTION

### Definitions

The term "screening" is understood herein as the examination or testing of a group of asymptomatic individuals pertaining to the general population, or a group of individuals suspected of having a disease, with the objective of discriminating healthy individuals from those who have or are suspected of having a disease. The term "subject having a disease" as used herein refers to those subjects diagnosed for a given disease. Preferably, the group of asymptomatic individuals includes those at high risk of developing the disease.

The term "diagnosis" as used herein refers to determining the presence or absence of a disease in a subject suspected of having the disease. A "diagnostic test" could be used to suggest or rule out the disease. Preferably, it may refer both to the process of attempting to determine and/or identify a possible disease in a subject, i.e. the diagnostic procedure, and to the opinion reached by this process, i.e. the diagnostic opinion. In a particular embodiment, the diagnosis to determine autoimmune and/or inflammatory disease (e.g. rheumatoid arthritis) in a subject, relates to the capacity to identify and classify patients having said autoimmune and/or inflammatory disease.

The term "subject suspected of having a disease" as used herein refers to a subject that presents one or more signs or symptoms indicative of said disease. A subject suspected of having a disease may also have one or more risk factors (i.e., a subject suspected of developing or at risk of developing a disease). It further encompasses an individual who has received a preliminary diagnosis but for whom a confirmatory test has not been done. Furthermore, it includes those individuals in remission. In a preferred embodiment, a subject suspected of having a disease does not encompass a subject at risk of developing a disease or a subject in remission.

The term "subject" as used herein refers to a mammalian subject. Preferably, it is selected from a human, companion animal, non-domestic livestock or zoo animal. For example, the subject may be selected from a human, dog, cat, cow, pig, sheep, horse, bear, and so on. In a preferred embodiment, said mammalian subject is a human subject.

The term "inflammatory rheumatic disease" as used herein refers to a disease of the locomotive apparatus and/or of the connective tissues in which inflammatory mechanisms play a relevant role. A rheumatic disease may affect the joints, bones, cartilage, tendons, ligaments and muscles. Preferably, this group of diseases include as illustrative non-limiting examples rheumatoid arthritis, juvenile rheumatoid arthritis, psoriatic arthritis, spondyloarthropathy (e.g. ankylosing spondylitis), lupus erythematosus, Sjogren syndrome, myositis, and scleroderma. A person skilled in the art will understand that this term will not include non-inflammatory rheumatic diseases such as degenerative diseases (e.g. arthrosis) or metabolic diseases (e.g. osteoporosis).

The term "early stage of progression" for an inflammatory rheumatic disease may refer to a stage of disease progression wherein there are no irreversible damages (e.g. no irreversible joint damages).

The term "early rheumatoid arthritis", "early RA" or "eRA" as used herein may refer to a subject diagnosed with rheumatoid arthritis, typically with symptom duration of ≤ 6 months, who has not received any first line therapy for the treatment of RA (i.e., corticosteroid or DMARD therapy) and whose conventional radiographs of hands and feet showed no structural damage.

The term "very early rheumatoid arthritis" or "veRA" as used herein may refer to a subject diagnosed with early RA with symptom duration of ≤ 3 months. More details on the classification of RA patients are provided in the ACR/EULAR classification criteria (Ann Rheum Dis 2010;69:1580-1588).

The term "disease monitoring" as used herein refers to determining the evolution of the disease, for example determining whether there is a remission of the disease, i.e., a reduction of clinical signs and/or symptoms characterizing the disease; or on the contrary whether there is disease progression or a relapse.

The term "response to a treatment" as used herein refers to the degree to which a treatment accomplishes the desired or projected outcomes, for instance the ability of a drug to achieve the desired prophylactic and/or therapeutic effect.

The term "treatment" encompasses both a prophylactic or therapeutic treatment. The term "therapeutic treatment" or "therapy" as used herein refers to bringing a body from a pathological state or disease back to its normal, healthy state. The term "prophylactic treatment" as used herein refers to preventing a pathological state.

The term "antibody-containing sample" or "antibody-containing biological sample" as used herein includes biological fluids, such as whole blood, serum, plasma, synovial fluid, cerebrospinal fluid, bronchial lavage, ascites fluid, bone marrow aspirate, pleural effusion, urine, as well as any tissue or any other bodily constituent that could contain antibodies or cells expressing thereof. Cells that express and secrete antibodies (B cells) are present in, and if appropriate may be isolated from, different tissues, organs and biological fluids from individuals. B cells may be found in the central or primary lymphoid organs that generate lymphocytes from immature progenitor cells, such as the thymus and the bone marrow. Alternatively, B cells may be found in secondary lymphoid tissue that provides the environment for the antigen to interact with the lymphocytes, such as the lymph nodes, the lymphoid follicles in tonsils, Peyer's patches, spleen, adenoids, skin, etc. that are associated with the mucosa-associated lymphoid tissue. It is noted that mononuclear cells in the spleen contain a higher percentage of IgG secreting cells. B cells may be found in from biological fluids such as blood, cerebrospinal, synovial or pleural fluids. Alternatively, lymphocytes may also be found in sites of chronic infection and/or inflammation.

The term "reference value" as used herein refers to a value determined in one or more samples, generally mean values of preferably a statistically significant number of samples, obtained from a reference or control population. Determination of the "reference value" is carried out under similar or identical conditions as the biological sample of the tested subject. Typically, said reference samples are from the same tissue or cell type and have undergone the same procedures for their obtaining and preservation. Alternatively; said "reference values" are preestablished values, generally mean values, in the control or reference population.

The term "kit" or "testing kit" denotes combinations of reagents and adjuvants required for an analysis. Although a test kit consists in most cases of several units, one-piece analysis elements are also available, which must likewise be regarded as testing kits.

The term "partially identical" as used herein refers to a sequence which is at least about 75%, at least about 80%, at least about 85%, at least about 90%, at least about 95%, at least about 96%, at least about 97%, at least about 98%, or at least about 99% identical to a reference sequence. In some embodiments, the partially identical sequence may be substantially identical to a reference sequence, that is at least about 95%, at least about 96%, at least about 97%, at least about 98%, or at least about 99% identical to said reference sequence. In one embodiment, the partially identical sequence is 100% identical to a reference sequence.

The term "identity" as used herein refers to an exact nucleotide-to-nucleotide or amino acid to amino acid correspondence of two polynucleotides or polypeptide sequences, respectively. Two or more sequences (polynucleotide or amino acid) can be compared by determining their "percent identity". The "percent identity" of two sequences, whether nucleic acid or amino acid sequences, is the number of exact matches between two aligned sequences divided by the length of the shorter sequence and multiplied by 100. Suitable programs for calculating the percent identity or similarity between sequences are well known in the art, such as the NCBI BLAST program, used for example with default parameters (http://www. ncbi. nlm. gov/cgi-bin/BLAST).

The term "Receiver Operating Characteristic (ROC) curves" as used herein refers to a graphical plot that illustrates the performance of a binary classifier system as its discrimination threshold is varied. The curve is created by plotting the true positive rate against the false positive rate at various threshold settings. The true positive rate is also known as sensitivity. The false positive rate is calculated as 1 - specificity. The ROC curve is thus a way of graphically displaying the true positive rate versus the false positive rate (sensitivity vs (1-specificity)) across a range of cut-offs and of selecting the optimal cut-off for clinical use. Accuracy expressed as the area under the ROC curve (AUC) provides a useful parameter for comparing test performance. An AUC approaching 1 indicates that the test is highly sensitive as well as highly specific whereas an AUC approaching 0.5 indicates that the test is neither sensitive nor specific. In general, a test is considered to be a suitable discriminator if the AUC is from 0.6 to 0.75, to have high discrimination capacity if the AUC is from 0.75 to 0.9 and to be an excellent discriminator if the AUC is from 0.9 to 1. For further details see for instance, Zweig MH and Campbell G, Clinical Chemistry 1993; 39:561-577 or Greiner et al. Preventive Veterinary Medicine 2000; 45:23-41.

### DETAILED DESCRIPTION

### An in vitro method for the screening of an autoimmune and/or inflammatory disease in a subject and methods related thereof

In a first aspect, the present invention relates to an *in vitro* method for the screening of a subject having, or suspected of having an inflammatory rheumatic disease comprising the following steps:
a) determining the levels of anti-CD26 IgG and/ or anti-CD26 IgM antibodies in an antibody-containing sample isolated from said subject;
b) comparing the levels in said antibody-containing sample with a reference value.

In a related aspect, the disclosure refers to an *in vitro* method for obtaining useful data for the diagnosis of an autoimmune and/or inflammatory disease in a subject, said method comprising the steps a) and b) of the first aspect.

In a further related aspect, the invention refers to an *in vitro* method for the diagnosis of an inflammatory rheumatic disease in a subject, said method comprising the steps a) and b) of the first aspect.

In an additional related aspect, the invention refers to an *in vitro* method for classifying subjects as i) healthy subjects or as ii) subjects having, or suspected of having an inflammatory rheumatic disease, said method comprising the steps a) and b) of the first aspect.

The first aspect and the above-described related aspects may herein be collectively referred as first aspect of the invention.

The term "inflammatory disease" is understood to be any disease where there is an excessive or altered inflammatory response that leads to inflammatory symptoms. Said inflammatory diseases include, without limitation, Addison's disease, acne vulgaris, alopecia areata, amyloidosis, ulcerations, aphthous stomatitis, arteriosclerosis, arthritis, osteoarthritis, rheumatoid arthritis, juvenile idiopathic arthritis, psoriatic arthritis, spondyloarthropathy (e.g. ankylosing spondylitis), bronchial asthma, Bechet's disease, Boeck's disease, intestinal inflammatory disease, Crohn's disease, choroiditis, ulcerative colitis, celiac's disease, cryoglobulinemia, macular degeneration, dermatitis, dermatitis herpetiformis, dermatomyositis, insulin dependent diabetes, juvenile diabetes, inflammatory demyelinating disease, Dupuytren contracture, encephalomyelitis, allergic encephalomyelitis, endophthalmia, allergic enteritis, autoimmune enteropathy syndrome, erythema nodosum leprosum, idiopathic facial paralysis, chronic fatigue syndrome, rheumatic fever, cystic fibrosis, gingivitis, glomerulonephritis, Goodpasture syndrome, Graves syndrome, Hashimoto's disease, chronic hepatitis, histiocytosis, regional ileitis, iritis, disseminated lupus erythematous, systemic lupus erythematous, cutaneous lupus erythematous, lymphogranuloma, infectious mononucleosis, miastenia gravis, transverse myelitis, primary idiopathic myxedema, nephrosis, obesity, sympathetic ophthalmia, granulomatous orchitis, pancreatitis, panniculitis, pemphigus vulgaris, periodontitis, polyarteritis nodosa, chronic polyarthritis, polymyositis, acute polyradiculitis, psoriasis, chronic obstructive pulmonary disease, purpura, gangrenous pioderma, Reiter's syndrome, diabetic retinopathy, rosacea, sarcoidosis, ataxic sclerosis, progressive systemic sclerosis, scleritis, sclerodermia, multiple sclerosis, disseminated sclerosis, acute anterior uveitis, vitiligo, Whipple's disease, diseases associated to AIDS, severe combined immunodeficiency and Epstein Barr's virus such as Sjogren's syndrome, osteoarticular tuberculosis and parasitic diseases such as leishmaniasis.

The term "autoimmune disease" as used herein refers to a non-malignant disease or disorder arising from and directed against an individual's own tissues. The autoimmune diseases herein specifically exclude malignant or cancerous diseases or conditions, especially excluding B cell lymphoma, acute lymphoblastic leukemia (ALL), chronic lymphocytic leukemia (CLL), Hairy cell leukemia and chronic myeloblastic leukemia. Examples of autoimmune diseases or disorders include, but are not limited to, inflammatory responses such as inflammatory skin diseases including psoriasis and dermatitis (e.g. atopic dermatitis); systemic scleroderma and sclerosis; responses associated with inflammatory bowel disease (such as Crohn's disease and ulcerative colitis); respiratory distress syndrome (including adult respiratory distress syndrome; ARDS); dermatitis; meningitis; encephalitis; uveitis; colitis; glomerulonephritis; allergic conditions such as eczema and asthma and other conditions involving infiltration of T cells and chronic inflammatory responses; atherosclerosis; leukocyte adhesion deficiency; rheumatoid arthritis; systemic lupus erythematosus (SLE); diabetes mellitus (e.g. Type I diabetes mellitus or insulin dependent diabetes mellitis); multiple sclerosis; Reynaud's syndrome; autoimmune thyroiditis; allergic encephalomyelitis; Sjorgen's syndrome; juvenile onset diabetes; and immune responses associated with acute and delayed hypersensitivity mediated by cytokines and T-lymphocytes typically found in tuberculosis, sarcoidosis, polymyositis, granulomatosis and vasculitis; pernicious anemia (Addison's disease); diseases involving leukocyte diapedesis; central nervous system (CNS) inflammatory disorder; multiple organ injury syndrome; hemolytic anemia (including, but not limited to cryoglobinemia or Coombs positive anemia); myasthenia gravis; antigen-antibody complex mediated diseases; anti-glomerular basement membrane disease; antiphospholipid syndrome; allergic neuritis; Graves' disease; Lambert-Eaton myasthenic syndrome; pemphigoid bullous; pemphigus; autoimmune polyendocrinopathies; Reiter's disease; stiff-man syndrome; Behcet disease; giant cell arteritis; immune complex nephritis; IgA nephropathy; IgM polyneuropathies; immune thrombocytopenic purpura (ITP) or autoimmune thrombocytopenia etc.

In the invention said autoimmune and/or inflammatory disease is an inflammatory rheumatic disease. Preferably, said inflammatory rheumatic disease is selected from the group consisting of rheumatoid arthritis, lupus erythematosus, Sjogren syndrome, juvenile rheumatoid arthritis, psoriatic arthritis and spondyloarthropathy (e.g. ankylosing spondylitis).

Elevated levels of TNFα are found in the synovial fluid of patients with rheumatoid arthritis, juvenile idiopathic arthritis, psoriatic arthritis and spondyloarthropathy (e.g. ankylosing spondylitis) and play an important role in both the pathologic inflammation and the joint destruction that are hallmarks of these diseases. Accordingly, in a preferred embodiment, said inflammatory rheumatic disease is selected from rheumatoid arthritis, juvenile rheumatoid arthritis, psoriatic arthritis and spondyloarthropathy (e.g. ankylosing spondylitis).

In another embodiment, said autoimmune and/or inflammatory disease is selected from the group consisting of rheumatoid arthritis, lupus erythematosus and Sjogren syndrome.

In a further preferred embodiment, said autoimmune and/or inflammatory disease is rheumatoid arthritis. In a particular embodiment, the method of diagnosis of the invention is a method for the differential diagnosis between rheumatoid arthritis and other diseases which present joint inflammation. For instance, it may be used for the differential between rheumatoid arthritis and one or more diseases selected from the group consisting of rheumatoid arthritis, lupus erythematosus, Sjogren syndrome, juvenile rheumatoid arthritis, psoriatic arthritis and spondyloarthropathy (e.g. ankylosing spondylitis).

Correlations of anti-CD26 levels with disease activity parameters, notably with tenderness of the joints, have been observed by the inventors (Example 6). Notably, according to Example 6.1 anti-CD26 levels of the three isotypes appear to correlate negatively with tender joints count (TJC) when considering the whole cohort. Also, the CD26 antigen used in the performed ELISA tests is not citrullinated suggesting that the detected auto-antibodies would recognize a non-citrullinated sCD26 form. Taken together this data may suggest that anti-CD26 antibodies appear in an early stage of the RA pathogenesis. Accordingly, in a preferred embodiment, said autoimmune and/or inflammatory disease is in an early, preferably in a very early, stage of progression.

**Step (a)** of the method under the first aspect of the invention requires the determination of anti-CD26 antibody levels in an antibody-containing sample isolated from a subject.

The term CD26 as used herein refers to human CD26 (UniProtKB - P27487), also known as dipeptidyl peptidase IV (DPP-IV). Its enzymatic activity is characterized by the release of an N-terminal dipeptide, Xaa-Yaa-|-Zaa-, from a polypeptide, preferentially when Yaa is Pro, provided Zaa is neither Pro nor hydroxyproline.

The term "CD26" encompasses, the 240 kDa homodimeric type II membrane glycoprotein composed of two 120 kDa subunits. (Mentlein, R., International Review of Cytology, 235: 165-213, 2004). Each of the homodimers of the membrane-bound form are characterized by a sequence consisting of positions 1 to 766 of SEQ ID NO:1 and the soluble form or "sCD26" is characterized by a sequence consisting of positions 39 to 766 of SEQ ID NO:1, as shown below in Table 1 below.

| Positions of SEQ ID NO:1 | Length | Description | UNIPROT identifier | SEQ ID NO |
|---|---|---|---|---|
| 1 - 766 | 766 | Dipeptidyl peptidase 4 membrane form | PRO_0000027213 | SEQ ID NO:1 |
| 39 - 766 | 728 | Dipeptidyl peptidase 4 soluble form | PRO_0000027214 | SEQ ID NO:2 |

SEQ ID NO: 1 (PRO_0000027213) has the following sequence:

The term "CD26" may also encompass sequences partially identical, preferably substantially identical to SEQ ID NO:1 or SEQ ID NO:2. These are collectively referred as "CD26 variants" and may be obtained by modifying SEQ ID NO:1 or SEQ ID NO:2 by substitution, deletion and/or addition.

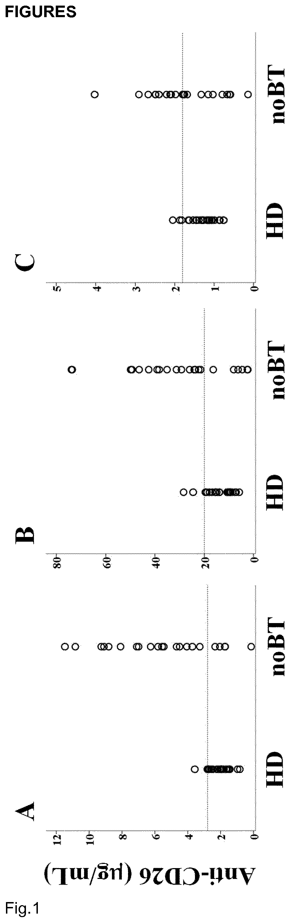

Deletion variants of CD26 and antigenic fragments of CD26 are characterized by containing at least one antigenic region or epitope. Illustrative non-limiting examples of CD26 epitopes are those defined by the binding of known anti-CD26 mAb which have been previously described, such as 4G8, 11H9, 1F7, 10F8A, 12E3B, 14D10, Tal, 5F8, 2F9, TA5.9, 18H3A, 16D4B, 9C11 (see anti-CD26 mAbs and their respective binding to CD26 deletion mutants in Figure 1 of Dong RP, et al., Mol Immunol. 1998, 35(1):13-21) and Anti-FLAG, 22C3, DS2-7, TA5.9, 1F7, 202-36, L272, Ta.1, BA5, CB.1, A3, A10, b10, f11 y C3 (see anti-CD26 mAbs and their respective binding to CD26 variants in Table 1 of Hühn J, et al.. Cell Immunol. 1999, 192(1):33-40; Torimoto Y, et al., Mol Immunol. 1992, 29(2):183-92). Granados et al. (Nat Commun 2014;5:3600) have recently described amino acid positions 145-154 of human CD26 as an MHC-I associated peptide (MIP) recognized by CD8 T cells.

In one embodiment, a CD26 variant may have one or more additional amino acid residues. Said additional amino acid residue(s) may be any amino acid, which may be either an L- and/or a D-amino acid, naturally occurring and otherwise. Preferably the amino acid is any naturally occurring amino acid such as alanine, cysteine, aspartic acid, glutamic acid, phenylalanine, glycine, histidine, isoleucine, lysine, leucine, methionine, asparagine, proline, glutamine, arginine, serine, threonine, valine, tryptophan or tyrosine.

However, the amino acid residue(s) may also be (a) modified or unusual amino acid(s). Examples of those are 2-aminoadipic acid, 3-aminoadipic acid, beta-alanine, 2-aminobutyric acid, 4-aminobutyric acid, 6-aminocaproic acid, 2-aminoheptanoic acid, 2-aminoisobutyric acid, 3-aminoisobutyric acid, 2-aminopimelic acid, 2,4-diaminobutyric acid, desmosine, 2,2'-diaminopimelic acid, 2,3-diaminopropionic acid, N-ethylglycine, N-ethylasparagine, hydroxylysine, allo-hydroxylysine, 3-hydroxyproloine, 4-hydroxyproloine, isodesmosine, allo-isoleucine, N-methylglycine, N-methylisoleucine, 6-N-Methyllysine, N-methylvaline, norvaline, norleucine or ornithine.

Additionally, the amino acid(s) may be subject to modifications such as posttranslational modifications. Examples of modifications include acetylation, amidation, blocking, formylation, γ-carboxyglutamic acid hydroxylation, glycosilation, methylation, phosphorylation, sulfatation and citrullination. Preferably, CD26 is not citrullinated.

If more than one additional amino acid residue is present in the polypeptide, the amino acid residues may be the same or different from one another. The CD26 polypeptide may be flanked by the amino acid residue(s) C-terminally, N-terminally, or C- and N-terminally.

In a particular embodiment of the invention said variant of CD26 is a variant having at least 70%, at least 80%, at least 90%, at least 95%, at least 99% identity to SEQ ID NO: 1 or SEQ ID NO: 2 which is derived by conservative substitutions. Conservative substitutions are those that take place within a family of amino acids that are related in their side chains and chemical properties and are well known to a person skilled in the art. Examples of such families are amino acids with basic side chains, with acidic side chains, with non-polar aliphatic side chains, with non-polar aromatic side chains, with uncharged polar side chains, with small side chains, with large side chains etc. In one embodiment, one conservative substitution is included in the polypeptide. In another embodiment, two conservative substitutions or less are included in the polypeptide. In a further embodiment, three conservative substitutions or less are included in the polypeptide.

CD26 may be obtained by recombinant protein techniques or by purification from human serum. Standard recombinant techniques in prokaryotic or eukaryotic host cells can be used to produce CD26. Suitable host cells include bacteria (e.g. E.coli), yeast, insect, or mammalian cells. A number of eukaryotic expression systems are available for expression of secreted proteins and membrane-bound proteins including those that contain unique post-translational modifications. The most common eukaryotic expression platforms currently include yeast (e.g., Pichia pastoris and Saccharomyces cerevisiae), insect cells (Spodoptera frugiperda or Trichoplusia ni), and mammalian cell systems (including a variety of transformed and/or genetically modified cell lines). Mammalian cell systems include but are not limited to simian, human, dog and rodent cells. Examples of human cells are PER.C6 cells (WO01/38362), MRC-5 (ATCC CCL-171), WI-38 (ATCC CCL-75), HEK-293 cells (ATCC CRL-1573), HeLa cells (ATCC CCL2), and fetal rhesus lung cells (ATCC CL- 160). Examples of non-human primate cells are Vero cells (ATCC CCL81), COS-1 cells (ATCC CRL-1650) or COS-7 cells (ATCC CRL-1651). Examples of dog cells are MDCK cells (ATCC CCL-34). Examples of rodent cells are hamster cells, such as BHK21-F, HKCC cells, or CHO cells. Any protein compatible expression system may be used to produce CD26. Suitable expression systems include transgenic animals described in Gene Expression Systems, Academic Press, eds. Fernandez et al., 1999. Moreover, any expression vectors known in the art can be used. Enzymes can be used to generate less than full-length proteins by enzymatic proteolysis of full-length or partial proteins.

Synthetic chemistry methods, such as solid-phase peptide synthesis, can also be used to synthesize CD26. In another particular embodiment, CD26 has been synthesized by means of any technique known in the art of peptide synthesis.

CD26 may be purified by means of any technique known in the art of protein purification. Exemplary techniques include ion-exchange chromatography, hydrophobic interaction chromatography, and immunoaffinity methods.

In a particular embodiment, CD26 is the soluble form of CD26 (sCD26), including variants thereof. In a preferred embodiment, sCD26 has been obtained by recombinant means and purified from a suitable host cell as described above. In a more preferred embodiment, said recombinant sCD26 has been purified from mouse myeloma cell line NS0, such as the commercially available sCD26 from RnD Systems, USA (1180-SE-010). In another more preferred embodiment, said recombinant sCD26 has been purified from baculovirus infected Sf9 cells, such as the commercially available sCD26 from Sigma-Aldrich, USA (D4943).

The methods of the present invention may be applied to samples from individuals of either sex, i.e. men or women, and at any age.

In some embodiments, said subject has not previously received or is not undergoing any biological treatment against said autoimmune and/or inflammatory disease, preferably has not previously received or is not undergoing any treatment against said autoimmune and/or inflammatory disease.

In other embodiments, said subject has previously received or is undergoing a treatment against said autoimmune and/or inflammatory disease, preferably, wherein said treatment is selected from the group consisting of
i. DMARDS;
ii. Anti-TNF alpha agents;
iii. Anti-CD20 agents; and
iv. Anti-IL6R agents and/or CTLA4-Fc fusion proteins.

The term "antibody-containing sample" has been defined above. These types of samples are routinely used in the clinical practice and a person skilled in the art will know how to identify the most appropriate means for their obtaining and preservation. Once a sample has been obtained, it may be used fresh, it may be frozen or preserved using appropriate means (e.g., as a formalin-fixed, paraffin-embedded tissue sample). When quantification is carried out by immunohistochemistry (IHC) analysis, typically thin sections of the biological sample immobilised on coated slides are used. These sections, when derived from a paraffin-embedded tissue samples, are deparaffinised and preferably treated so as to retrieve the protein to be detected. The detection can be carried out in individual samples or in tissue microarrays.

Preferably, this antibody-containing sample is whole blood, serum or plasma, more preferably is serum.

The quantification of antibody levels under step a) of the first aspect of the invention may be expressed in terms of "antibody concentration" and/or "antibody titer". The term "antibody titer" and "antibody concentration" may be used herein interchangeably.

The term "antibody concentration" as used herein refers to the total amount of antibody (protein) in solution without regard to function. Antibody concentration can be estimated using either a general protein assay or an immunoglobulin-specific method.

The term "antibody titer" as used herein refers to the functional dilution (or working concentration) of an antibody sample that is necessary to achieve a minimum level of specific detection in a given assay method. The exact minimum acceptable value is established by the researcher, but it is usually defined by reference to a statistically significant signal-to-noise ratio. Antibody titer is generally considered an assay-specific measurement.

Preferably, the determination of antibody levels under step a) of the first aspect of the invention is performed by an immunoglobulin-specific method. Various types of immunoassays are known to one skilled in the art for the quantitation of proteins of interest. These methods are based on the use of affinity reagents, which may be any antibody or ligand specifically binding to the target protein, which is preferably labeled.

For example, western blotting or immunoblotting techniques allow comparison of relative abundance of proteins separated by an electrophoretic gel (e.g., native proteins by 3-D structure or denatured proteins by the length of the polypeptide). Immunoblotting techniques use antibodies (or other specific ligands in related techniques) to identify target proteins among a number of unrelated protein species. They involve identification of protein target via antigen-antibody (or protein-ligand) specific reactions. Proteins are typically separated by electrophoresis and transferred onto a sheet of polymeric material (generally nitrocellulose, nylon, or polyvinylidene difluoride). Dot and slot blots are simplified procedures in which protein samples are not separated by electrophoresis but immobilized directly onto a membrane.

In some embodiments of the invention, immunoglobulin quantification may be performed by quantifying the corresponding immunoglobulin expressing cells (e.g. B cells producing anti-CD26 antibodies of a given isotype). This may be possible for instance by immunohistochemistry assays, where proteins are detected directly in cells of a tissue. The role of B cells and autoantibodies in tissue destructive events of autoimmune diseases is emerging, and thereby increasing interest in identifying the presence and location of autoreactive B cells and autoantibody secreting plasma cells. For visualization and analysis of the autoreactive B cells, the antigen of interest is selected and produced and purified from native or recombinant sources. Appropriate labelling (e.g. biotinylation) of the purified antigen and subsequent use in immunohistochemistry with sections from tissue under analysis permits detection of the autoreactive B cells and plasma cells. Double staining with cell-specific markers or for the presence of intracellular Ig allows characterization of the cell or determination of Ig isotype of the autoantibody produced by the individual autoreactive B -cell. With this technique, identification as well as quantification of autoreactive cells within tissues may be performed; it is also possible to analyze the spatial relation to residual cells or other infiltrating cells of the target organ. For illustrative purposes, a non-limiting example on how this may be carried out is described in Wharen-Herlenius and Salomonsson (Methods Mol Med. 200,136:19-24).

Quantification of immunoglobulin expressing cells may also be possible in any kind of antibody-containing biological fluids using techniques well known in the art such as flow cytometry and fluorescence-activated cell sorting (FACS). A specific selection and quantification of antibody-secreting cells can be performed, using one of the many methods described in the literature, usually on the basis of the expression of specific antibody isotypes and/or cell surface markers on their surface. In particular, various technologies for the purification of antibody-secreting cells from human samples make use of different means and conditions for positive or negative selection. These cells are more easily and efficiently selected by physically separating those expressing cell surface markers specific for cells that express and secrete antibodies (e.g. B cells). Specific protocols can be found in the literature (see Callard R and Kotowicz K "Human B-cell responses to cytokines" in Cytokine Cell Biology: A practical Approach. Balkwill F. (ed.) Oxford University Press, 2000, pg.17-31).

Traditionally, quantification of proteins in solution has been carried out by immunoassays on a solid support. Said immunoassay may be for example an enzyme-linked immunosorbent assay (ELISA), a fluorescent immunosorbent assay (FIA), a chemiluminescence immunoassay (CIA), or a radioimmunoassay (RIA), an enzyme multiplied immunoassay, a solid phase radioimmunoassay (SPROA), a fluorescence polarization (FP) assay, a fluorescence resonance energy transfer (FRET) assay, a time-resolved fluorescence resonance energy transfer (TR-FRET) assay, a surface plasmon resonance (SPR) assay. Multiplex and any next generation versions of any of the above, such as bead-based flow-cytometry immunoassays (e.g., based on the Luminex xMAP technology) are specifically encompassed. In a particular embodiment, said immunoassay is an ELISA assay, a cell-ELISA or any multiplex version thereof.

Other methods that can be used for quantification of proteins are techniques based on mass spectrometry (MS) such as liquid chromatography coupled to mass spectrometry (LC / MS), described for example in US2010/0173786, or tandem LC-MS / MS (WO2012/155019, US2011/0039287, M. Rauh, J Chromatogr B Analyt Technol Biomed Life Sci 2012 February 1, 883-884. 59-67) and the use of arrays of peptides, proteins or antibodies and multiplex versions of the above techniques, as well as the next generation of such techniques and combinations thereof.

In a particular embodiment, anti-sCD26 antibody levels present in an antibody-containing suspension sample are determined by an immunoassay wherein sCD26 has been coated on a solid phase support. In another embodiment, anti-CD26 antibody levels present in an antibody-containing suspension sample are determined by an immunoassay wherein a cell expressing CD26 is found on a solid phase support (e.g. cell-ELISA). Below it is generally described a method for the determination of anti-sCD26 antibodies of a selected isotype.

sCD26, or any antigenic fragments thereof, is bound to the solid support. It can be adsorbed or chemically coupled to a solid phase support. Any means known in the art for immobilizing a protein or peptide to a solid support can be used. sCD26 can be either covalently or non-covalently bound to the solid phase support by techniques such as covalent bonding via an amide or ester linkage or adsorption. It can also be bound using binding pairs such as biotin and avidin or antibody and antigen. After sCD26 is affixed to the solid phase, the solid phase support can be incubated with a blocking solution (containing a blocking protein such as bovine serum albumin) to reduce non-specific adsorption of antibodies in a test sample to the support surface.

Various solid phase supports can be used, including but not limited to glass, polystyrene, polypropylene, nitrocellulose, dextran or other materials. Suitable forms of the solid phase supports include beads, microparticles, tubes, fabrics or plates formed from or coated with these materials. In a preferred embodiment the solid support comprises microtiter wells, such as a 96-well microtiter plate.

A detection antibody is used to assess the anti-sCD26 antibody bound to the solid phase support. The detection antibody used for this purpose can be isotype-specific, e.g. IgA, IgD, IgE, IgG, or IgM. The isotype-specific detection antibody can be labeled and detected. Antibody sub-isotypes may also be determined.

The detection antibody may be labeled. A label can be any composition which is detectable. Any analytical means known in the art can be used for determining or detecting the detection antibody. These means include the use of spectroscopy, chemistry, photochemistry, biochemistry, immunochemistry, or optics. The label can be, for example, an enzyme (e.g., horseradish peroxidase (HRP), alkaline phosphatase, beta-galactosidase, and others commonly used in an ELISA), a radiolabel (e.g., ³H, ¹²⁵I, ³⁵S, ¹⁴C, or ³²P), a chemiluminescent compound (e.g. luciferin, and 2,3-dihydrophthalazinediones, luminol, etc.), a fluorescent dye (e.g., fluorescein isothiocyanate, Texas red, rhodamine, etc.), or any other dye known in the art.

The label may be coupled directly or indirectly (e.g., via binding pairs such as biotin and avidin) to the detection antibody according to methods well known in the art. As indicated above, a wide variety of labels may be used. The choice of label may depend on sensitivity required, ease of conjugation with the compound, stability requirements, available instrumentation, or disposal provisions.

Detection antibodies can be detected or quantified by any suitable method known in the art. A label on an antibody can be detected by a gamma counter if the label is a radioactive gamma emitter, or by a fluorimeter, if the label is a fluorescent material. In a preferred embodiment, the detection antibody is detected using a HRP-conjugated species-specific immunoglobulin. Any known substrate of HRP can be used. Non-limiting examples are ABTS (2,2'-Azinobis [3-ethylbenzothiazoline-6-sulfonic acid]-diammonium salt), OPD (o-phenylenediamine dihydrochloride) and TMB (3,3',5,5'-tetramethylbenzidine). Each of these substrates yields a reaction product that can be detected optically with a maximum absorbance at 410 nm and 650 nm, at 492 nm and at 450 nm, respectively.

Results of the assay may be qualitative or quantitative. The amount of label associated with the support can be compared with positive and negative controls in order to determine the presence of anti-sCD26 antibodies. The controls are typically run concomitantly with the sample to be tested. A positive control can be a serum containing antibodies that are immunoreactive with sCD26. A negative control can be a serum that does not contain antibodies that are immunoreactive with the sCD26. For quantitation, a standard curve using known quantities of anti-sCD26 antibody can be generated and/or used.

Antibodies for use as positive controls may be produced using all, or fragments of, the amino acid sequence of sCD26. "Antibody" as used herein includes intact immunoglobulin molecules, as well as fragments thereof, such as Fab, F(ab')₂, and Fv, which are capable of binding an epitope of the target protein (e.g. sCD26). Any type of antibody known in the art can be generated to bind specifically to an epitope of sCD26. Monoclonal or polyclonal antibodies can be made as is well known in the art.

Any technique for purifying antibodies available in the art can be used. For example, antibodies can be purified by known methods such as affinity separation using protein A, high pressure liquid chromatography on reverse phase alkylated silica gel, or gel filtration. Antibodies can also be passed over a solid phase to which sCD26 is bound. The anti-sCD26 antibodies will bind to the sCD26 bound to the solid support and the contaminants can be washed away. The bound antibodies can be eluted, for example, with a buffer having a high salt concentration.

The particular parameters employed in the assay for anti-CD26 antibody levels determination can vary widely depending on various factors such as the concentration of antibody in the sample, the nature of the sample, the type of immunoassay employed and the like. Optimal conditions can be readily established by those of ordinary skill in the art. Typical assay conditions include a temperature range of about 4° C to about 45° C and a pH value range of about 5 to 9. Incubation times can also vary widely depending upon the nature of the assay, and generally range from about 0.1 minute to about 24 hours. A broad variety of buffers, for example TRIS-buffered saline, may be employed, and other reagents such as salt to enhance ionic strength, proteins such as serum albumin, stabilizers, and non-ionic detergents may also be included. Exemplary conditions for total immunoglobulins and anti-CD26 autoantibodies of the IgG, IgM and IgA isotypes are given in Example 1.

### The ratio between anti-CD26 IgG antibody levels and total IgG antibody levels

As an alternative or in addition to determining the levels of anti-CD26 IgG antibodies, the methods of the invention may include the determination of the ratio between anti-CD26 IgG antibody levels and total IgG antibody levels (herein after referred as "anti-CD26 IgG ratio").

Total antibody levels of a particular isotype may be determined by an immunoassay in a solid support wherein, instead of sCD26 as described above, an anti-human antibody of the relevant isotype (e.g., IgM, IgG or IgA) generally of another animal species is bound to the solid support, e.g., a polyclonal rabbit anti-human IgM, anti-IgG or anti-IgA.

Accordingly, the ratio between anti-CD26 IgG antibody levels and total IgG antibody levels (i.e., the "anti-CD26 IgG ratio") is obtained herein by dividing the anti-CD26 IgG antibody levels by the total IgG antibody levels.

Accordingly, in another particular embodiment, the methods of the invention comprise the following steps:
a) determining the levels of anti-CD26 IgG antibodies and/or the anti-CD26 IgG ratio in an antibody-containing sample isolated from said subject;
b) comparing the levels and/or ratio in said antibody-containing sample with a reference value.

In a further particular embodiment, the methods of the invention comprise the following steps:
a) determining the levels of anti-CD26 IgM antibodies and/or the anti-CD26 IgG ratio in an antibody-containing sample isolated from said subject;
b) comparing the levels and/or ratio in said antibody-containing sample with a reference value.

In a preferred embodiment, the method under the first aspect of the invention and any related aspect as described above, comprises the following steps:
a) determining the levels of anti-CD26 IgG antibodies in an antibody-containing sample isolated from said subject;
b) comparing the levels in said antibody-containing sample with a reference value.

In **step b)**, the levels and/or ratio determined in said biological sample are compared with reference values.

A reference value represents a threshold value of the relevant biomarker (e.g. anti-CD26 IgG antibody levels) to which a subject's measured levels may be compared. The reference level can be selected based on the intended purpose of the test or assay being performed on the subject. For example, a reference level may be determined for purposes of identifying subjects affected by an autoimmune and/or inflammatory disease while the same or different reference level may be used for monitoring the progression or severity of disease in a subject or the responsiveness to a treatment.

The selection of an appropriate reference level for a given test or assay is within the level of skill in the art. The choice of the reference value is not absolute. For example, a relatively low value may advantageously be used to reduce the incidence of false negatives, but may also increase the likelihood of false positives. Accordingly, as for other screening, diagnosis or monitoring techniques, the reference value may be based on a number of factors, including but not limited to cost, the benefit of early diagnosis and treatment, the invasiveness of follow-up diagnostic methods for individuals that have false positive results, the intended purpose of the test or assay, the limits of accurate detection in the particular sample type, the prevalence and average or of the given biomarker on the relevant population, the desired level of statistical significance of the results, the level of quantification desired in terms of disease risk, presence, progression, or severity, and other factors that are routinely considered in designing screening assays.

A threshold value or cut-off level may be determined for use in the methods of the invention. The term "reference value" as used herein also encompasses a threshold or cut-off level. A variety of statistical and mathematical methods for establishing the threshold or cutoff level for a particular biomarker are known in the prior art and may be selected, for example, based on data from Receiver Operating Characteristic (ROC) plots.

One of skill in the art will appreciate that these threshold or cutoff expression levels can be varied, for example, by moving along the ROC plot for a particular biomarker or combinations thereof, to obtain different values for sensitivity or specificity thereby affecting the overall assay performance. The best cut-off refers to the value obtained from the ROC plot for a particular biomarker that produces the best sensitivity and specificity. Sensitivity and specificity values are calculated over the range of thresholds (cut-offs). Thus, the threshold or cut-off values can be selected such that the sensitivity and/or specificity are at least about 70 %, and can be, for example, at least 75 %, at least 80 %, at least 85 %, at least 90 %, at least 95 %, at least 96 %, at least 97 %, at least 98 %, at least 99 % or at least 100% in at least 60 % of the patient population assayed, or in at least 65 %, 70 %, 75 %, 80 %, 85 %, 90 %, 95 % or preferably 100% of the population assayed.

In some embodiments of the methods under the first and related aspects of the invention, said reference value corresponds to the respective anti-CD26 antibody levels or ratio reference value in healthy subjects. The term "healthy subjects" as used herein refers to an appropriate unaffected population.

In the methods under the first and related aspects of the invention, said disease is an inflammatory rheumatic disease and said reference value corresponds to the respective anti-CD26 antibody levels or ratio reference value of a rheumatic disease which is not an inflammatory rheumatic disease, such as osteoarthritis.

When comparing the subject's levels with said reference or control value (e.g., the mean or median levels or a cut-off value), the degree of variation of the levels in the tested sample with respect to a reference value may be for instance by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more.

Alternatively or in addition, subjects having more than about 1.2, 1.3, 1.4, 1.5, 2, 3, 4, 5, 8, 10 or 20 fold higher biomarker concentration than the reference value (e.g., the mean or median levels or a cut-off value) for an appropriate unaffected population as measured under similar or identical conditions may be identified as having, or suspected of having an autoimmune and/or inflammatory disease.

Preferably, this variation is statistically significant. The term "significant" or "statistically significant" when referring to differences between the test sample values and the control or reference value, relates to the condition when using the appropriate statistical analysis the probability of the groups being the same is less than 5%, e.g. p<0.05. In other words, the probability of obtaining the same results on a completely random basis is less than 5 out of 100 attempts. A person skilled in the art will know how to choose the appropriate statistical analysis. Typically, the appropriate statistical analysis is determined based on whether the variable under study has a normal distribution, for instance by using the test of Kolmogorov-Smirnov and on whether there is homoscedasticity, which is determined for instance with the Levene test. Preferably, in those cases where there is a normal distribution and homoscedasticity, a parametric model such as t-test or ANOVA test is used; and where at least one of these two requirements is not accomplished then a non-parametric model such as Mann-Whitney U test or Kruskal-Wallis test is generally used to compare continuous variables (not categorized). For categorized variables Chi-square test or Fisher exact test are typically performed. For multiple comparisons to a single cohort Dunnett's t-test is typically used.

In preferred embodiments of the methods of the invention, an increase of the relevant anti-CD26 antibody levels as defined above (e.g., anti-CD26 IgG antibody levels and/or the anti-CD26 IgG ratio and/or anti-CD26 IgM antibody levels) in the subject sample with regard to the respective reference value is indicative of disease. Preferably, wherein said reference value corresponds to the respective reference value in healthy subjects.

The methods under the first aspect and related aspects as defined above may further comprise determining the anti-CD26 antibody levels of the IgA isotype in said antibody-containing sample.

Accordingly, in some embodiments of the invention, the determined anti-CD26 antibody levels are selected from the group consisting of:
i. the anti-CD26 IgG antibody levels and/or the anti-CD26 IgG ratio;
ii. the anti-CD26 IgM antibody levels;
iii. the IgG levels and/or ratio recited in i) and the anti-CD26 IgM antibody levels;
iv. the IgG levels and/or ratio recited in i) and the anti-CD26 IgA antibody levels; and
v. the IgG levels and/or ratio recited in i) and the anti-CD26 IgM, and anti-CD26 IgA antibody levels.
wherein for each of the defined groups an increase of any and preferably all of the antibody levels or ratio in the subject sample with regard to said reference value is indicative of disease and wherein said reference value corresponds to the respective reference value in healthy subjects.

In a preferred embodiment, said method comprises:
a) determining the levels of anti-CD26 IgG antibodies in an antibody-containing sample isolated from said subject;
b) comparing the anti-CD26 IgG antibody levels in said antibody-containing sample with a reference value; and
further comprises:
c) determining the anti-CD26 antibody levels of the IgM and/or IgA isotype in said antibody-containing sample;
d) comparing the anti-CD26 IgM and/or IgA antibody levels in said antibody-containing sample with a reference value;
wherein an increase of the anti-CD26 IgG antibody levels value and of the anti-CD26 IgM and/or anti-CD26 IgA antibody levels in the subject sample with regard to the respective reference value is indicative of disease and wherein said reference value corresponds to the respective reference value (e.g. the mean levels) in healthy subjects.

In a preferred embodiment of any of the above, the increase of said anti-CD26 antibody levels with respect to said reference value is of at least 1.2x, preferably of at least 1.3x, more preferably of at least 1.4x, even more preferably of at least 1.5x, and most preferably of at least 2x. In a more preferred embodiment, the increase for IgG is of at least 1.3x, the increase for IgM is of at least 1.4x, and the increase of IgA is of at least 1.3x.

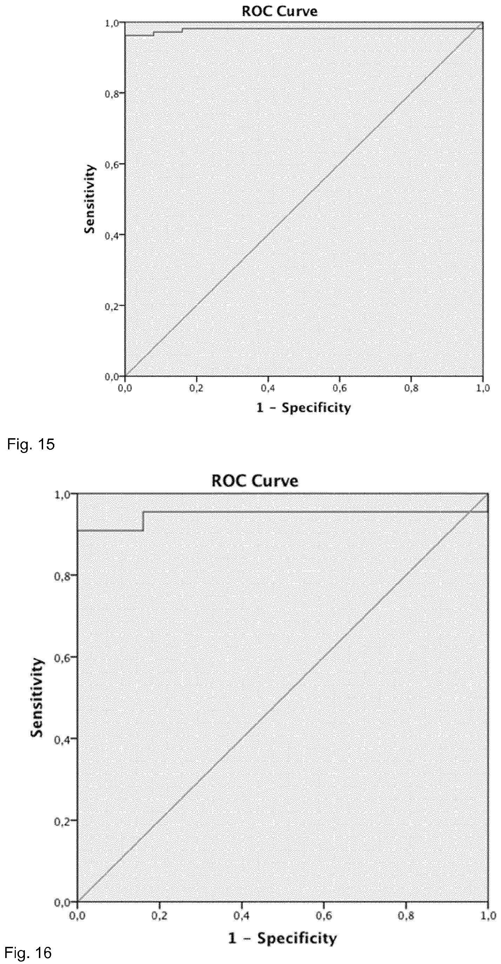

The method according to the first and related aspects may further comprise the determination of IgA total antibodies levels, which have shown a high discrimination value between healthy donors and the whole cohort (see Figure 15, AUC of 0.979) and between healthy donors and patients undergoing DMARDS but no biological therapy (Figure 16, AUC: 0.947).

The method according to the first and related aspects may further comprise the determination of other biomarkers associated to said autoimmune and/or inflammatory disease, preferably, said biomarkers are selected from the group consisting of anti-citrullinated protein antibodies (ACPA), rheumatoid factor (RF) antibodies, anti-mannose binding lectin (MBL) antibodies, erythrocyte sedimentation rate (ESR), C-reactive protein (CRP), platelet count, hemoglobin levels and hematocrit.

In a particular embodiment, these are selected from the list consisting of ESR, CRP concentration levels, anti-citrullinated protein antibodies (ACPA), rheumatoid factor auto-antibodies and anti-mannose binding lectin (MBL) auto-antibodies which have been previously been used as diagnostic markers. Further details are provided in Gupta B et al. J Autoimmun. 2006. 27: 125-133, Afzal Net al. Clin Lab. 2011. 57: 895-899, Takizawa Y et al. Ann Rheum Dis. 2006. 65: 1013-1020, Vossenaar ER et al. Arthritis Res Ther. 2004. 6: R142-R150 and EP0175270 which are incorporated herein by reference.

In a particular embodiment, the method of the invention further comprises determining anti-citrullinated protein antibodies (ACPA) levels in said subject. The results in Example 10 suggest that anti-CD26 antibodies participate in RA pathogenesis in an independent way than ACPA, and may serve for earlier diagnosis, in particular in those subjects which are ACPA negative. In another particular embodiment, optionally in combination with any of the above, the methods of the invention as described herein may further comprise the determination of clinical parameters. Signs and/or symptoms which presence/absence may be determined are: morning stiffness, joint involvement (i.e. any swollen or tender joint on examination) including number (e.g. arthritis of three or more joint areas) and type of joints (e.g. large joints, small joints, or hand joint involvement), symmetric arthritis, rheumatoid nodules, and radiographic changes (see Table 1 of Rindfleisch and Muller (American Family Physician 2005, 72(6), 1037-1047), and ACR/EULAR classification criteria (Ann Rheum Dis 2010; 69:1580-1588).

In a particular embodiment, optionally in combination with any of the above, the methods of the invention as described herein further comprise the determination of biomarkers and clinical parameters as described herein. Preferably, these include one or more, preferably all, of the following i) determination of joint involvement, ii) determination of anti-citrullinated protein antibodies (ACPA) and/or rheumatoid factor (RF) antibodies levels, and iii) erythrocyte sedimentation rate (ESR) and/or C-reactive protein (CRP) determination. Preferably, the method of the invention is combined with the current 2010 ACR/EULAR classification criteria, provided in Table 3 of Ann Rheum Dis 2010; 69:1580-1588 and reproduced below:

**Table 3 The 2010 American College of Rheumatology/European League Against Rheumatism classification criteria for RA**

| | **Score** |
|---|---|
| Target population (Who should be tested?): Patients who | |
| 1) have at least 1 joint with definite clinical synovitis (swelling)* | |
| 2) with the synovitis not better explained by another disease† | |
| Classification criteria for RA (score-based algorithm: add score of categories A-D; a score of ≥6/10 is needed for classification of a patient as having definite RA)‡ | |

| A. Joint involvement§ | |
|---|---|
| 1 large joint¶ | 0 |
| 2-10 large joints | 1 |
| 1-3 small joints (with or without involvement of large joints)** | 2 |
| 4-10 small joints (with or without involvement of large joints) | 3 |
| > 10 joints (at least 1 small joint)†† | 5 |

| B. Serology (at least 1 test result is needed for classification)‡‡ | |
|---|---|
| Negative RF *and* negative ACPA | 0 |
| Low-positive RF *or* low-positive ACPA | 2 |
| High-positive RF *or* high-positive ACPA | 3 |

| C. Acute-phase reactants [at least 1 test result is needed for classification]§§ | |
|---|---|
| Normal CRP *and* normal ESR 0 | 0 |
| Abnormal CRP *or* normal ESR 1 | 1 |

| D. Duration of symptoms¶¶ | |
|---|---|
| <6 weeks | 0 |
| ≥6 weeks | 1 |

| | |
|---|---|
| *The criteria are aimed at classification of newly presenting patients. In addition, patients with erosive disease typical of rheumatoid arthritis (RA) with a history compatible with prior fulfilment of the 2010 criteria should be classified as having RA. Patients with long-standing disease, including those whose disease is inactive (with or without treatment) who, based on retrospectively available data, have previously fulfilled the 2010 criteria should be classified as having RA. †Differential diagnoses differ in patients with different presentations, but may include conditions such as systemic lupus erythematosus, psoriatic arthritis and gout. If it is unclear about the relevant differential diagnoses to consider, an expert rheumatologist should be consulted. ‡Although patients with a score of less than 6/10 are not classifiable as having RA, their status can be reassessed and the criteria might be fulfilled cumulatively over time. § Joint involvement refers to any swollen or tender joint on examination, which may be confirmed by imaging evidence of synovitis. Distal interphalangeal joints, first carpometacarpal joints and first metatarsophalangeal joints are excluded from assessment. Categories of joint distribution are classified according to the location and number of involved joints, with placement into the highest category possible based on the pattern of joint involvement. ¶'Large joints' refers to shoulders, elbows, hips, knees and ankles. **'Small joints' refers to the metacarpophalangeal joints, proximal interphalangeal joints, second to fifth metatarsophalangeal joints, thumb interphalangeal joints and wrists. ††In this category, at least one of the involved joints must be a small joint; the other joints can include any combination of large and additional small joints, as well as other joints not specifically listed elsewhere (eg, temporomandibular, acromioclavicular, sternoclavicular, etc.). ‡‡Negative refers to international unit (IU) values that are less than or equal to the upper limit of normal (ULN) for the laboratory and assay; low-positive refers to IU values that are higher than the ULN but three of less times the ULN for the laboratory and assay: high-positive refers to IU values that are more than three times the ULN for the laboratory and assay. When rheumatoid factor (RF) information is only available as positive or negative, a positive result should be scored as low-positive for RF. §§Normal/abnormal is determined by local laboratory standards. ¶¶Duration of symptoms refers to patient self-report of the duration of signs or symptoms of synovitis (eg, pain, swelling, tenderness) of joints that are clinically involved at the time of assessment, regardless of treatment status. ACPA, anti-citrullinated protein antibody; CRP, C-reactive protein; ESR, erythrocyte sedimentation rate. | |

A "confirmed diagnosis of rheumatoid arthritis" or "definite rheumatoid arthritis" may be based on the confirmed presence of synovitis in at least one joint, absence of an alternative diagnosis better explaining the synovitis, and achievement of a total score of 6 or greater (of a possible 10) from the individual scores in four domains: number and site of involved joints (range 0-5), serological abnormality (range 0-3), elevated acute-phase response (range 0-1) and symptom duration (two levels; range 0-1). The meaning of these parameters is as defined in ACR/EULAR classification criteria (Ann Rheum Dis 2010; 69:1580-1588).

The term "joint involvement" as used herein refers to any swollen or tender joint on examination, which may be confirmed by imaging evidence of synovitis. Distal interphalangeal joints, first carpometacarpal joints and first metatarsophalangeal joints are typically excluded from assessment. Categories of joint distribution are generally classified according to the location and number of involved joints, with placement into the highest category possible based on the pattern of joint involvement. The term "large joints" as used herein, refers to shoulders, elbows, hips, knees and ankles. The term "small joints" as used herein, refers to the metacarpophalangeal joints, proximal interphalangeal joints, second to fifth metatarsophalangeal joints, thumb interphalangeal joints and wrists.

Preferably, the method of the invention further comprises storing the results of the method in a data carrier. The results of the method of the invention include the determined UNR gene product expression values, the determination of the subject as having good or poor prognosis, the determination of the subject as having short-term or long-term outcome after surgery, and any other determinations according to any of the methods of the invention, as well as any mathematical or statistical treatment thereof. Said data carrier may be a paper sheet or preferably a computer readable medium. As used herein, "a computer readable medium" can be any apparatus that may include, store, communicate, propagate, or transport the results of the determination of the method of the invention. The medium can be an electronic, magnetic, optical, electromagnetic, infrared, or semiconductor system (or apparatus or device) or a propagation medium.

The methods of the present invention might be implemented by a computer. Therefore, a further aspect of the invention refers to a computer implemented method, wherein the method is any of the methods disclosed herein or any combination thereof. It is noted that any computer program capable of implementing any of the methods of the present invention or used to implement any of these methods or any combination thereof, also forms part of the present invention.

It is also noted that any device or apparatus comprising or carrying a computer program capable of or, for implementing any of the methods of the present invention or any combination therefore, is included as forming part of the present specification.

### An in vitro method of disease monitoring and/or monitoring responsiveness to a treatment in a subject having or suspected to have an inflammatory rheumatic disease disease and methods related thereof

In another aspect, the invention relates to an *in vitro* method of disease monitoring and/or monitoring responsiveness to a treatment in a subject having or suspected to have an inflammatory rheumatic disease, said method comprising the steps a) and b) of the first aspect of the invention. In a particular embodiment, an increase of the anti-CD26 IgG and/ or anti-CD26 IgM antibody levels and/or the anti-CD26 IgG antibody levels /total IgG antibody levels ratio in the subject sample with regard to the reference value is indicative of disease.

In a related aspect, the invention refers to an *in vitro* method for the classification of a subject as responder to a treatment to an inflammatory rheumatic disease, said method comprising the steps a) and b) of the first aspect of the invention. In a particular embodiment, an increase of the anti-CD26 IgG and/ or anti-CD26 IgM antibody levels and/or the anti-CD26 IgG antibody levels /total IgG antibody levels ratio in the subject sample with regard to said reference value is indicative of lack of response.

For any of the above aspects, said reference value may correspond to a reference value (e.g., the relevant anti-CD26 antibody mean levels or ratio) in healthy subjects. It may also correspond to said anti-CD26 antibody levels determined in an appropriate sample isolated from said patient in an earlier moment in time, such as around diagnosis and/or before treatment.

Preferred features and embodiments are as defined above for other aspects of the invention.

### A method of treating a subject identified with a method comprising steps a) and b) of the first aspect and related second medical uses

In a further aspect, the present description relates to a method of treating a subject having an autoimmune and/or inflammatory disease, said method comprising identifying the subject to be treated by a method comprising the steps a) and b) of the first aspect of the invention and
c) treating said subject suffering or suspected to suffer from an autoimmune and/or inflammatory disease.

In a particular embodiment, an increase of any of the anti-CD26 IgG and/ or anti-CD26 IgM antibody levels and/or the anti-CD26 IgG antibody levels /total IgG antibody levels ratio in the subject sample with regard to said reference value is indicative of disease.

Said reference value may correspond to a reference value (e.g., the relevant anti-CD26 antibody mean levels or ratio) in healthy subjects.

Said therapies may include one or more of disease-modifying anti-rheumatic drugs (DMARDs); nonsteroidal anti-inflammatories (NSAIDs); biological therapies; corticosteroids; and JAK kinase inhibitors (such as tofacitinib).

The term "biological therapies" or "biologics" as used herein refers to "biologic response modifiers" (BRM) which are drugs which stimulate or suppress the immune system. Biological therapies have successfully been used in the treatment of immune-mediated conditions such as RA and may be broadly divided as i) anti-TNF alpha biologics (also referred as TNFi biologics, e.g., adalimumab, cetolizumab pegol, etanercept, golimumab or infliximab) and ii) non-TNF alpha biologics, including anti-CD20 agents (e.g. rituximab), anti-IL6R agents (e.g. tocilizumab) or CTLA4-Fc fusion proteins (e.g. abatacept).

NSAIDs including but are not limited to Cox-2 inhibitors, diclofenac, ibuprofen, naproxen, celecoxib, mefenamic acid, etoricoxib, indometacin and aspirin. Corticosteroids include but are not limited to triamcinolone, cortisone, prednisone, and methylprednisolone. Disease-modifying antirheumatic drugs (DMARDs), include but are not limited to auranofin, choroquine, cyclosporine, cyclophosphamide, gold preparations, hydroxychloroquine sulphate, leflunomide, methotrexate, penicillamine, sodium aurothiomalate and sulfasalazine. Illustrative non-limiting examples of therapeutic antibodies used for autoimmune and/or inflammatory diseases are provided in Table 1 of Chan and Carter, Nature Reviews on Immunology, 2010, 10, 301-315.

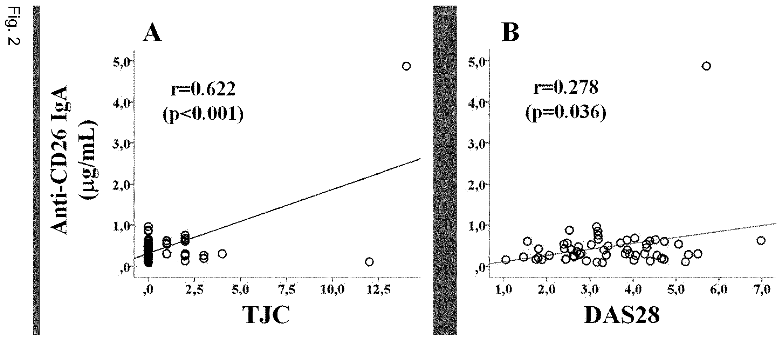

Singh et al., (Arthritis & Rheumathology, 2016, 68(1), 1-16) provides a guideline for the treatment of RA from the American College of Rheumatology. Table 1 (partially reproduced below) provides a summary of current RA therapeutic treatments. In particular treatment recommendations for patients with symptomatic early RA are provided in the table of Figure 2 in page 8 of Singh et al.

| Drug category | Descriptions |
|---|---|
| Methotrexate | Used either oral or subcutaneous (a DMARD). |
| DMARDs§ | Traditional/conventional DMARDs including HCQ, LEF, MTX, or SSZ (excludes azathioprine, cyclosporine, minocycline, and gold), it does not include tofacitinib, which is considered separately.¶ |
| DMARD monotherapy | Most often defined as the use of MTX monotherapy, but may also be SSZ, HCQ, or LEF. |
| Double DMARD therapy | MTX+SSZ, MTX+HCQ, SSZ+HCQ, or combinations with LEF. |
| Triple DMARD therapy | MTX+SSZ+H CQ. |
| DMARD combination therapy | Double or triple traditional/conventional DMARD therapy. |
| Tofacitinib | Oral synthetic small molecule. |
| Biologics | TNFi biologic or non-TNF biologic (excludes anakinra).§ |
| TNFi biologics | Adalimumab, certolizumab pegol, etanercept, golimumab, or infliximab. |
| Non-TNF biologics | Abatacept, rituximab, or tocilizumab (excludes anakinra).§ |
| Low-dose glucocorticoid | ≦ 10 mg/day of prednisone (or equivalent). |
| High-dose glucocorticoid | > 10 mg/day of prednisone (or equivalent) and up to 60 mg/day with a rapid taper.# |
| Short-term glucocorticoid | < 3 month treatment. |

| | |
|---|---|
| * ACR = American College of Rheumatology; RA = rheumatoid arthritis; PROMIS = Patient-Reported Outcomes Measurement Information System; EULAR = European League Against Rheumatism; DAS = Disease Activity Score; DMARD = disease-modifying antirheumatic drug; AST = aspartate aminotransferase; ALT = alanine aminotransferase; HBV = hepatitis B virus; HCV = hepatitis C virus; NYHA = New York Heart Association; HCQ = hydroxychloroquine; LEF = leflunomide; MTX = methotrexate; SSZ = sulfasalazine; TNFi = tumor necrosis factor inhibitor; COBRA = Combinatietherapie Bij Reumatoide Artritis. † Any of the ACR recommended disease activity measures may be chosen, as described in ref. 16. ‡ New classification criteria for RA (ACR/EULAR collaborative initiative) were published in 2010 (82), the definition of established RA is based on the 1987 ACR RA classification criteria, since the 2010 ACR RA classification allows a much earlier diagnosis. § Anakinra was considered but not included in these guidelines due to its infrequent use in RA and lack of new data since 2012, ¶ Azathioprine, cyclosporine, minocycline, and gold were considered but not included in these guidelines due to their infrequent use in RA and/ or lack of new data since 2012. # Regimen based on that described in the COBRA study (153). | |

In a particular embodiment, said method of treating comprises the use of one or more agents selected from the group consisting of:
i. DMARDs, preferably methotrexate, and/or leflunomide;
ii. anti-TNF alpha agents;
iii. anti-CD20 agents;
iv. anti-IL6R agents; and
v. CTLA4-Fc fusion proteins, such as abatacept.

In a related aspect, the description refers to a drug selected from the list consisting of:
i. DMARDS, preferably methotrexate, and/or leflunomide;
ii. Anti-TNF alpha agents;
iii. Anti-CD20 agents; and
iv. Anti-IL6R agents and/or CTLA4-Fc fusion proteins, such as abatacept;
for use in a method of treating a subject suffering or suspected of suffering from an autoimmune and/or inflammatory disease, wherein said subject has been selected by using a method according to any of the methods described herein.

In another related aspect, it concerns the use of a drug selected from the list consisting of:
i. DMARDS, preferably methotrexate, and/or leflunomide;
ii. Anti-TNF alpha agents;
iii. Anti-CD20 agents; and
iv. Anti-IL6R agents and/or CTLA4-Fc fusion proteins, such as abatacept;
in the manufacturing of a medicament for the treatment of a subject suffering or suspected of suffering from an autoimmune and/or inflammatory disease, wherein said subject has been selected by using a method according to any of the methods described herein.

Preferred features and embodiments are as defined above for other aspects of the invention.

### An in vitro method for determining the stage of progression of an autoimmune and/or inflammatory disease in a subject and methods related thereof

In further aspect, the description relates to an *in vitro* method for determining the stage of progression of an autoimmune and/or inflammatory disease in a subject, said method comprising the steps a) and b) of the first aspect of the invention.

In a related aspect, the description refers to an *in vitro* method of determining a treatment for a subject suffering from an autoimmune and/or inflammatory disease according to its stage of progression comprising the steps a) and b) of the first aspect of the invention.

In a particular embodiment of any of the above aspects, an increase of any of the anti-CD26 IgG and/ or anti-CD26 IgM antibody levels and/or the anti-CD26 IgG antibody levels /total IgG antibody levels ratio in the subject sample with regard to said reference value is associated with early stage of progression.

Also, for any of the above aspects, said reference value may correspond to a reference value (e.g., the relevant anti-CD26 antibody mean levels or ratio) in healthy subjects. It may also correspond to a reference value (e.g., the relevant anti-CD26 antibody mean levels or ratio) in patients having or suspected to have an anti-inflammatory and/or autoimmune disease, preferably an inflammatory rheumatic disease, more preferably the same anti-inflammatory and/or autoimmune disease.

Preferred features and embodiments are as defined above for other aspects of the invention.

In a preferred embodiment, optionally in combination with any of the embodiments described herein, it relates to an *in vitro* method for determining the stage of progression of an autoimmune and/or inflammatory disease in a subject, said method comprising the steps a) and b) of the first aspect, wherein an increase of the anti-CD26 IgG antibody levels in the subject sample with regard to said reference value is associated with early stage of progression;
wherein said reference value corresponds to the mean anti-CD26 IgG antibody levels in healthy subjects.

In another preferred embodiment, optionally in combination with any of the embodiments described herein, it relates to an in *vitro* method of determining a treatment for a subject suffering from an autoimmune and/or inflammatory disease according to its stage of progression comprising the steps a) and b) of the first aspect,
wherein an increase of the anti-CD26 IgG antibody levels in the subject sample with regard to said reference value is indicative of early stage of progression;
wherein said reference value corresponds to the mean anti-CD26 IgG antibody levels in healthy subjects.

### Use of in vitro determined anti-CD26 antibody levels as biomarkers of an autoimmune and/or inflammatory disease

Use of *in vitro* determined anti-CD26 IgG and/ or anti-CD26 IgM and/or the anti-CD26 IgG antibody levels/total IgG antibody levels ratio, optionally in combination with *in vitro* determined anti-CD26 IgA antibody levels, in an antibody-containing sample isolated from a subject for the screening, diagnosis, and/or monitoring of an autoimmune and/or inflammatory disease; and/or for monitoring the effectiveness of a treatment in an autoimmune and/or inflammatory disease.

Preferred features and embodiments are as defined above for other aspects of the invention.

### Kit and uses thereof

In an additional aspect, the description relates to a kit comprising reagents for determining anti-CD26 antibody levels according to step a) of the first aspect of the invention. These reagents may be as described under the first aspect of the invention for various types of immunoassays.

In some embodiments, the kit includes a purified or synthesized sCD26 polypeptide or an antigenic fragment thereof (which may be labelled or not) and one or more ancillary reagents. In other embodiments, the kit includes a suitable host cell (e.g. a mammalian cell) expressing CD26 and one or more ancillary reagents. CD26, including sCD26 and fragments thereof have been described under the first aspect of the invention. In these embodiments, when anti-CD26 antibodies are quantified sCD26 or a cell expressing CD26 is typically used for capturing purposes and a secondary or detection antibody for detection purposes. In still further embodiments, when anti-CD26 immunoglobulin expressing cells are quantified, e.g. by IHC or cytometry, the kit typically includes labelled sCD26 for detection purposes and one or more ancillary reagents.

### Kit anti-CD26 IgG isotype

In a particular embodiment, said kit comprises reagents for determining anti-CD26 antibody levels of the IgG isotype in an antibody-containing sample isolated from a subject, wherein said kit comprises:
a) a reagent for determining the levels of anti-CD26 antibodies of the IgG isotype;
b) optionally, instructions for the use of said reagent in determining the anti-CD26 antibody levels of the IgG isotype in an antibody-containing sample isolated from said subject.

Preferably, a) corresponds to:
i. CD26, an antigenic fragment thereof or a cell expressing any thereof;
ii. a reagent for determining the levels of CD26-captured antibodies of the IgG isotype, such as a labelled anti-IgG affinity reagent (e.g., a labelled anti-IgG antibody).

In a preferred embodiment, said kit further comprises:
c) a reagent for determining the levels of anti-CD26 antibodies of the IgA and/or IgM isotype;
d) optionally, instructions for the use of said reagent in determining the anti-CD26 antibody levels of the IgA and/or IgM isotype in said antibody-containing sample.

Preferably, c) corresponds to:
iii. a reagent for determining the levels of CD26-captured antibodies of the IgA and/or IgM isotype, such as a labelled anti-IgM affinity reagent and/or a labelled anti-IgA affinity reagent (e.g., a labelled anti-IgA antibody and/or a labelled anti-IgM antibody).

### Kit anti-CD26 IgM isotype

In another particular embodiment, said kit comprises reagents for determining anti-CD26 antibody levels of the IgM isotype in an antibody-containing sample isolated from a subject, wherein said kit comprises:
a) a reagent for determining the levels of anti-CD26 antibodies of the IgM isotype;
b) optionally, instructions for the use of said reagent in determining the anti-CD26 antibody levels of the IgM isotype in an antibody-containing sample isolated from said subject.

Preferably, a) corresponds to:
i. CD26, an antigenic fragment thereof or a cell expressing any thereof;
ii. a reagent for determining the levels of CD26-captured antibody of the IgM isotype, such as a labelled anti-IgM affinity reagent (e.g., a labelled anti-IgM antibody).

In a preferred embodiment, said kit further comprises:
c) a reagent for determining the levels of anti-CD26 antibodies of the IgA and/or IgG isotype;
d) optionally, instructions for the use of said reagent in determining the anti-CD26 antibody levels of the IgA and/or IgG isotype in said antibody-containing sample.

Preferably, c) corresponds to:
iii. a reagent for determining the levels of CD26-captured antibodies of the IgA and/or IgG isotype, such as a labelled anti-IgA affinity reagent and/or a labelled anti-IgG affinity reagent (e.g., a labelled anti-IgA antibody and/or a labelled anti-IgG antibody).

Preferably, said kit comprises a solid support or surface which is coated with i). The solid support can include any support known in the art on which a protein of this disclosure can be immobilized. In some embodiments, said solid supports are microtiter well plates, slides (e.g., glass slides), chips (e.g., protein chips, biosensor chips, such as Biacore chips), microfluidic cartridges, cuvettes, beads (e.g., magnetic beads, xMAP® beads) or resins.

Said reagent for determining the levels of antibody of the IgM, IgG and/or IgA isotype may be a secondary or detection antibody. Preferably, the antibody of the IgM, IgG and/or IgA isotype is a human antibody and the secondary or detection antibody is an anti-human antibody. Secondary or detection antibodies in the present invention can include, e.g., an anti-human IgA antibody, an anti-human IgG antibody, or an anti-human IgM antibody. These antibodies can be monoclonal or polyclonal antibodies. Also, these can be derived from any mammalian organism, including mice, rats, hamsters, goats, camels, chicken, rabbit, and others. Secondary or detection antibodies can be conjugated to any suitable detection means such as enzymes (e.g., horseradish peroxidase (HRP), alkaline phosphatase (AP), luciferase, and the like) or dyes (e.g., colorimetric dyes, fluorescent dyes, fluorescence resonance energy transfer (FRET)-dyes, time-resolved (TR)-FRET dyes, and the like). Other suitable labels have been described herein above. In some embodiments, the secondary or detection antibody is a polyclonal goat-anti-human IgG (anti-human IgM or anti-human IgA antibody, respectively depending on the isotype to be determined), which is HRP-conjugated.

Any of the above embodiments on the kit, may further comprise:
e) a reagent for determining the levels of total antibodies of the IgM, IgG and/or IgA;
f) optionally, instructions for the use of said reagent in determining the levels of total antibodies of the IgM, IgG and/or IgA isotype in said antibody-containing sample.

Preferably, e) corresponds to:
iv. a reagent for capturing antibodies of the IgM, IgG and/or IgA isotype, such as an anti- IgM, IgG and/or IgA affinity reagent (e.g., an anti-IgM, anti-IgG and/or anti-IgA antibody)
v. a reagent for determining the levels of captured antibodies of the IgM, IgG and/or IgA isotype, such as a labelled anti- IgM, IgG and/or IgA affinity reagent (e.g., a labelled anti-IgM, anti-IgG and/or anti-IgA antibody);

Typically, the reagent in iv) and v) are polyclonal antibodies against the relevant isotype originating from different animal species. Preferably, these are anti-human antibodies. (e.g. a polyclonal rabbit anti-human IgG and a polyclonal goat anti-human IgG, respectively). These may also be monoclonal antibodies, with the proviso that, these bind to different epitopes and there is no steric interference.

Any of the above embodiments on the kit may further comprise:
- a reagent for determining the levels of anti-citrullinated protein antibodies (ACPA); and
- optionally, instructions for the use of said reagent in determining the levels of ACPA in said antibody-containing sample.

This reagent may comprise one or more antigens in citrullinated proteins, such as those typically included in anti-cyclic citrullinated peptide (anti-CCP) antibody assays (Johansson et al. Arthritis Res Ther. 2016; 18: 127; Nijenhuis S, et al.,Clin Chim Acta 2004, 350:17-34; van Venrooij W. J. and Zendman A. J. W. Clin Rev Allergy Immunol. 2008, 34(1): 36-39). These citrullinated antigens are not particularly limited and may be endogenous (e.g. histone-4-derived citrullinated peptides (HCP1 and HCP2) or exogenous (e.g. viral citrullinated peptides (VCP) derived from Epstein-Barr-virus nuclear antigen (EBNA)1 and EBNA2 (VCP1 and VCP2). The sequences of the specifically mentioned VCPs and HCPs have been described in Johansson et al. Arthritis Res Ther. 2016; 18: 127.

Preferably, said kit comprises a solid surface or support, which is coated with iv). This solid surface or support may be as described herein above. This surface may or not be the same, which is coated with i). Preferably, the surface coated with i) and the surface coated with iv) are separate surfaces.

Ancillary reagents typically used in an immunoassay, such as a solid support immunoassay, can include, e.g., an immobilization buffer, an immobilization reagent, a dilution buffer, a secondary or detection antibody, a detection reagent, a blocking buffer, a washing buffer, a detection buffer, a stop solution, a system rinse buffer, and a system cleaning solution which are well known by a person skilled in the art.

Preferred features and embodiments are as defined above for other aspects of the invention. In particular, an example of immunoassay to determine anti-CD26 antibody levels is provided under the first aspect of the invention.

In still an additional aspect, the invention relates to the use of a kit of the preceding aspect for the screening, diagnosis, and/or monitoring of an autoimmune and/or inflammatory disease; and/or for monitoring the effectiveness of a treatment in an antibody-containing sample isolated from a subject.

Preferred features and embodiments are as defined above for other aspects of the invention.

### Total IgA levels as biomarker for screening, diagnosis, monitoring of an inflammatory and/or autoimmune disease and related aspects.

As provided in Example 5, IgA total antibodies levels, have shown a high discrimination value between healthy donors and the whole cohort of patients treated with different therapies (see Figure 15, AUC of 0.979) and between healthy donors and patients undergoing DMARDS but no biological therapy (Figure 16, AUC: 0.947).

Accordingly, in a further aspect, the invention relates to an *in vitro* method for the screening, for obtaining useful data for the diagnosis, for the diagnosis or for the monitoring of a subject having, or suspected of having an inflammatory rheumatic disease comprising the following steps:
a) determining the levels of total antibodies of the IgA isotype (total IgA antibody levels) in an antibody-containing sample isolated from said subject;
b) comparing the levels in said antibody-containing sample with a reference value.

In a particular embodiment, an increase of the total IgA antibody levels in the subject sample with regard to said reference value is indicative of disease.

Said reference value may correspond to a reference value for total IgA antibody levels (e.g., the mean or median levels or a calculated cut-off value) in healthy subjects. It may also correspond to said total IgA antibody levels determined in an appropriate sample isolated from said patient in an earlier moment in time, such as around diagnosis and/or before treatment.

Total IgA antibody levels (e.g. total human IgA antibody levels) may be determined by any method well-known in the art. These are typically quantified by using an immunoassay, including in particular an immunoassay in a solid support as described in detail under the first aspect of the invention wherein the solid support is coated with an anti-IgA antibody (e.g. an anti-human IgA antibody).

In a further aspect, the present description relates to a method of treating a subject having an autoimmune and/or inflammatory disease, said method comprising identifying the subject to be treated by a method comprising the steps of:
a) determining the levels of total antibodies of the IgA isotype (total IgA antibody levels) in an antibody-containing sample isolated from said subject;
b) comparing the levels in said antibody-containing sample with a reference value; and
c) treating said subject suffering or suspected to suffer from an autoimmune and/or inflammatory disease.

In a particular embodiment, an increase of the total IgA antibody levels in the subject sample with regard to said reference value is indicative of disease.

Said reference value may correspond to the total IgA antibody levels reference value (e.g., the mean or median levels or a cut-off value) in healthy subjects.

In an additional aspect, the description relates to a kit comprising reagents for determining total antibody levels of the IgA isotype in an antibody-containing sample isolated from a subject, wherein said kit comprises:
a) a reagent for determining the levels of total antibody levels of the IgA isotype;
b) optionally, instructions for the use of said reagent in determining the total IgA antibody levels in an antibody-containing sample isolated from said subject.

In a particular embodiment, said kit further comprises:
c) a reagent for determining the levels of an anti-CD26 antibody of the IgG isotype;
d) optionally, instructions for the use of said reagent in determining the anti-CD26 antibody levels of the IgG isotype in an antibody-containing sample isolated from said subject.

In another particular embodiment, optionally in combination with the embodiment above, said kit further comprises:
e) a reagent for determining the levels of anti-CD26 antibody of the IgA and/or IgM isotype;
f) optionally, instructions for the use of said reagent in determining the anti-CD26 antibody levels of the IgA and/or IgM isotype in said antibody-containing sample.

In still an additional aspect, the invention relates to the use of a kit for the screening, diagnosis, and/or monitoring of an inflammatory rheumatic disease; and/or for monitoring the effectiveness of a treatment in an antibody-containing sample isolated from a subject, wherein said kit is as described in the preceding aspect.

Preferred embodiments and features of any of these aspects are as described above for the other aspects of the invention.

It is contemplated that any embodiment discussed in this specification with respect to one aspect of the invention applies to other aspects of the invention as well and viceversa, more specifically it can be implemented with respect to any method, kit, and use of the invention described herein. It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine experimentation, numerous equivalents to the specific procedures described herein. Such equivalents are considered to be within the scope of this invention and are covered by the claims.

The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the terms "consists of" and "consists essentially of". As used herein, the phrase "consisting essentially of" limits the scope of a claim to the specified materials or steps and those that do not materially affect the basic and novel characteristic(s) of the claimed invention. As used herein, the phrase "consisting of excludes any element, step, or ingredient not specified in the claim except for, e.g., impurities ordinarily associated with the element or limitation.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

A person skilled in the art will generally understand that a value includes the standard deviation of error for the device or method being employed to determine the value. This may be emphasized herein by the use of words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, 10, 11, 12, 13, 14 or 15%. Preferably the term "about" means exactly the indicated value (± 0%).

The following examples serve to illustrate the present invention and should not be construed as limiting the scope thereof.

### EXAMPLES

### Example 1.- Material and Methods

### Study Design

110 patients from the Rheumatology Service (Hospital Meixoeiro-CHUVI) were recruited in a cross-sectional case-control study. Patients fulfilled the American College of Rheumatology (ACR)/EULAR criteria of 2010 (Aletaha D et al. Arthritis Rheum. 2010. 62: 2569-2581 and were on different therapies, including biological therapies (BT). The only exclusion criterion was the patient's decision not to be included in the study. A group of 25 healthy donors was also recruited. All the procedures described were performed according to clinical ethical practices of the Spanish and European Administrations and approved by the local ethics committee. Written informed consent was obtained from all participants.

Disease activity was previously assessed in this group of patients by the DAS-28 index, which takes into account the number of tender joints (TJC), swollen joints (SJC), erythrocyte sedimentation rate (ESR) and Patient Global Assessment (PGA) of disease activity, scored by a numeric rating scale (NRS 0-100). Erythrocyte sedimentation rate (ESR), C-reactive protein (CRP), haemoglobin and platelet concentration levels were also recorded as RA activity markers. The DAS-28 index and how to determine it is well known to a person skilled in the art, see for instance J.S. Smolen, et al. Arthritis Rheum 1995, 38: 38-43, the content of which is included herein by reference. All patients also completed the HAQ (Stanford Health Assessment Questionnaire: Fries J, Spitz PW, Young DY. The dimensions of health outcomes: the health assessment questionnaire, disability and pain scales. J Rheumatol 1982, 9: 789-93), which is a self-report functional status (disability) measure. The results on the assessed disease activity parameters have been published before by the inventors (Cordero OJ et al. PLoS One. 2015. 10(7):e0131992) and the content of this document is included herein by reference.

### Different group of patient's defined according to the undergoing therapies

The patients under study were classified in different groups according to the therapies they were receiving, which may have different effect on the antibody values. The cohort was divided into four groups according to the mechanism of action of the different therapies:
a) Disease-Modifying Anti-Rheumatic Drugs (DMARDs; methotrexate and/or leflunomide);
b) anti-TNF-alpha agents (adalimumab, etanercept, infliximab, golimumab, certolizumab),
c) anti-CD20 mAb (rituximab), and
d) anti-IL6R mAb (tocilizumab) or Ig-CTLA4 (abatacept). In this latter subgroup both therapies showed a similar immunological response, so patients' results were grouped together.

### Smoking history

Patients' smoking history was evaluated at inclusion. Current smokers were those reporting active smoking. Past smokers were all patients who had stopped smoking before the first examination at inclusion. Non-smokers reported no history of smoking at any time.

### Biological samples

For serum collection, peripheral venous blood extracted with BD SST™ II *Advance* tubes was allowed to clot at room temperature and centrifuged at 2,000 x g for 15 min. Serum was stored at -80 °C until use. Blood cells were collected using TransFix® Vacuum Blood Collection Tubes (Cytomark, Buckingham, UK) and stored at 4 °C until use.

### Measurement of DPP-IV Enzyme Activity and Soluble CD26 Protein

Both techniques have been described previously [Cordero OJ et al. PLoS One. 2015. 10(7):e0131992, Cuchacovich M et al. Clin Exp Rheumatol. 2001. 19: 673-80, Ellingsen T et al. Scand J Immunol. 2007. 66: 451-7]. Since previous studies [Cordero OJ et al. Immunobiology. 1997. 197:522-33, De Chiara L et al. Dis Markers. 2009. 27:311-6. doi: 10.3233/DMA-2009-0679, Lambeir AM et al. Crit Rev Clin Lab Sci. 2003. 40: 209-94] have shown slight but not statistically significant differences in both levels of sCD26 and DPP-IV activity according to gender or age, values from controls and RA patients were not matched for these two parameters.

### Enzyme-linked immunosorbent assays for total immunoglobulins and anti-CD26 autoantibodies.

Concentration of both total and anti-CD26/DPP-IV IgA, IgG and IgM titers in sera of patients was determined by ELISA using 96-well cultured plates coated with rDPP-IV (0.5 µg/mL) (Sigma-Aldrich Spain or RnD Systems, USA) or with polyclonal rabbit anti-human IgA, anti-IgG and anti-IgM (2 µg/mL) (DakoCytomation, Denmark) prepared in PBS pH 7.4 and blocked overnight with PBS 0.5% BSA. Plates were incubated with different dilutions of serum for 1 h at 37 °C and then washed four times with PBS 0.05% Tween20. Goat anti-human IgM (µ-chain), anti IgG (Fab-specific) and IgA (a-specific)-peroxidase conjugates (all from Sigma-Aldrich) were used to detect captured antibodies together with standard OPD substrate (Sigma-Aldrich) following manufacturer's instructions. Absorbance at 450 nm was registered using a BioRad Plate reader (BioRad, Madrid, Spain). Concentrations were calculated from calibration curves constructed with affinity-purified human IgA, IgG or IgM.

### ACPA Test

Elia™ CCP Test (Thermo Scientific/Phadia, Sweden) was used to analyse whether anti-CD26 autoantibodies in RA were antibodies against a citrullinated CD26. Briefly, the test was used to bind the anti-CCP antibodies from patients' sera to the plate containing CPP antigens, according to the manufacturers' instructions. The remaining serum was recovered as negative-ACPA fraction. After washing the Elia-CCP plate to avoid unspecific binding, ACPA were eluted from the plate with Elution buffer (0.1 M Gly-HCI pH 2.7) and this buffer neutralized with 1 M Tris-HCI pH 9 to obtain the positive-ACPA fraction. Both fractions were then checked using the in-house anti-CD26 ELISA described above. In the same way, this latter ELISA was used to obtain a negative anti-CD26 Ab fraction (recovery of patients' serum after antibody (Ab) binding to the plate-bound CD26 Ag) and a positive-antiCD26 Ab fraction (obtained after washing and elution of the bound Ab) and both fractions analyzed using the Elia™ CCP test.
Sera from three RA patients showing high levels of anti-CD26 IgG isotype and three healthy donors were chosen. In all the ELISAs, only reagents to detect the IgG isotype (described above) were used.

### Statistical Analysis

All analyses were parametric. The ANOVA test was carried out to compare variables among the four groups of patients with or without biological therapies. The post-hoc Scheffé test was used for variables with homogeneous variances and the post-hoc Dunnett's C test was used for variables without homogeneous variances. Dunnett's t test was performed to compare each group with a control group, either the group without biological therapy or the healthy donor group. Student's t test was also used to compare variables between two groups. Statistical analyses were carried out using the SPSS version 20 software (SPSS, Chicago IL, USA).

### Example 2.- Anti-CD26/DPP-IV autoantibody levels and their correlations with serum DPP-IV activity and sCD26 concentrations in the healthy donor cohort

Autoantibodies against CD26 have been described in healthy donors and proposed to participate in antigen clearance via bile secretion (Cuchacovich M et al. Clin Exp Rheumatol. 2001. 19: 673-80). With our in-house ELISA, mean serum titers (n=25) of total immunoglobulin concentrations for the three isotypes under study are found coherent with the expected values, 2.37 ±1.24 mg/mL for IgM, 2.28 ±0.27 mg/mL for IgA and 12.28 ±2.79 mg/mL for IgG (Gonzalez-Quintela A et al. Clinical and Experimental Immunology. 2007. 151: 42-50). Mean anti-CD26 serum autoantibody titers are also quite similar to those previously published (Snir O et al. Arthritis Rheum. 2010. 62: 44-52. 10.1002/art.25036 [doi]), 13.51 ±5.62 µg/mL for IgM, 1.28 ±0.27 µg/mL for IgA and 2.14 ±0.64 µg/mL for IgG, being the present values slightly lower for the IgM and IgA isotypes when compared to previous results published by Cuchacovich et al. (Cuchacovich M et al. Clin Exp Rheumatol. 2001. 19: 673-80) probably due to the fact that we used recombinant human antigen instead purified CD26 from human serum. However, comparing Cuchacovich et al. published ratios of anti-CD26 autoantibody/total Ig isotype with our data (7.12 for IgM, 0.57 for IgA and 0.19 for IgG), our results are more coherent with the expected values of total Igs titers.
Importantly, none of the presented serum titer values, including the reduced anti-CD26 IgA isotype levels, nor their ratios, correlate with either serum DPP-IV activity or sCD26 concentrations in this cohort of healthy donors suggesting that if their function is related to antigen clearance, their effect on sCD26 levels is undetectable.

### Example 3.- Anti-CD26/DPP-IV and total antibody levels in RA patients undergoing different therapies

### 3.1 Anti-CD26/DPP-IV and total antibody levels in the whole cohort

Analyzing our whole cohort of RA patients (n=106) we found higher levels of both total and anti-CD26 Ig titers. Total immunoglobulin concentrations for the three isotypes are 4.33 ±3.79 mg/mL for IgM, 5.61 ±1.77 mg/mL for IgA and 21.81 ±15.35 mg/mL for IgG (Lambeir AM et al. Crit Rev Clin Lab Sci. 2003. 40: 209-94). Anti-CD26 autoantibody concentrations are 27.97 ±19.09 µg/mL for IgM, 2.01 ±1.34 µg/mL for IgA and 5.87 ±3.13 µg/mL for IgG, and auto-antibody/total isotype ratios are 17.10 for IgM, 0.43 for IgA and 0.75 for IgG. Thus, although all values of total Igs increase two-fold overall, it is interesting to note that ratios for IgA isotypes are lower compared to healthy donors whereas this was not the case for the IgM and particularly for IgG isotype ratio, being higher (next to 4-fold) and significantly different compared to healthy donors (p<0.001; ANOVA test), in addition to all mean values for total or auto-antiCD26 isotype titers (p<0.001 for most of them with the Student's T-test). These results point to the usefulness of anti-CD26 antibody titers as biomarker of RA.

### 3.2 Anti-CD26/DPP-IV and total antibody levels according to therapy

The anti-CD20 therapy group may serve as control. Therapeutic mAbs that target the CD20 antigen on B cells are successfully used in the clinic for the depletion of B cells to treat various forms of cancer and autoimmune diseases, and thus by its mechanism of action reduce the levels of immunoglobulins in general. Anti-CD20 therapy was shown to reduce the levels of total IgG and particularly IgM, as expected, but had no effect on total IgA (Table 2) compared to the group without biological therapy. The observed decrease does not reach the antibody levels seen in healthy donors, however. On the contrary, the other biological therapies were shown to raise the levels of total IgG and IgM with the anti-IL6R/Ig-CTLA4 group being the only showing a slightly lower level of IgA (Table 2).

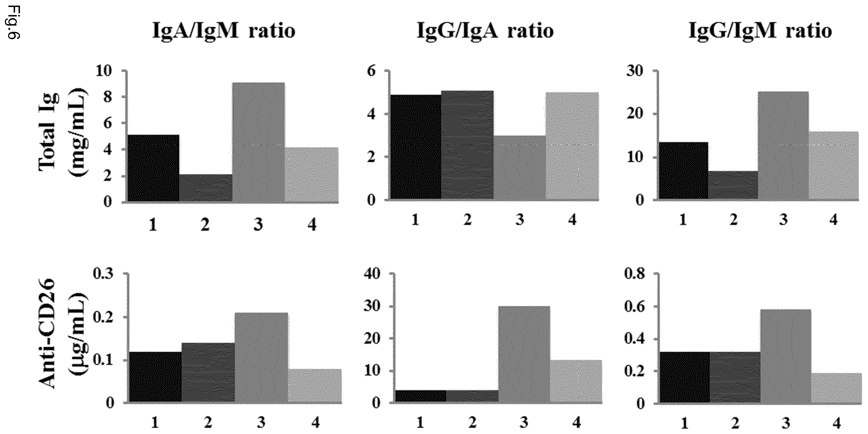

However, the effect of different therapies on anti-CD26 auto-Ab levels was different. The IgG isotype was not affected by any therapy; anti-CD20 strongly reduces only the IgM isotype and finally the IgA isotype is affected by both the anti-TNF and anti-IL6R/Ig-CTLA4 therapies but in contrary directions (Table 2). As a consequence, ratios for each of the three isotypes, are quite different compared to healthy subjects, particularly IgM for anti-TNF and anti-IL6R/Ig-CTLA4 BT, IgA for anti-CD20 and IL6R/Ig-CTLA4 BT, and IgG for NoBT and IL6R/Ig-CTLA4 BT, underlying the specificity of our results. Most of these differences were statistically significant (see Table 2). The same can be concluded when checking the isotype ratios (A/M, G/A, G/M) for total or for auto-Ab titers: statistically significant differences between patients and donors in some ratios (Table 3) and important differences among the therapies (Fig 6, check the different behaviour of the G/A ratio in total and auto-Ab titers for the anti-CD20 therapy).

**Table 2. Levels of total and anti-CD26 Ig isotypes in RA patients under different therapies**

| | **HC (n=25)** | | **No BT (n=22)** | | **Anti-TNFα BT (n=58)** | | **Anti-CD20 BT (n=11)** | | **Anti-IL6R/Ig-CTLA4 BT (n=15)** | |
|---|---|---|---|---|---|---|---|---|---|---|
| | Mean ± SD | CI (95%) | Mean ± SD | CI (95%) | Mean ± SD | CI (95%) | Mean ± SD | CI (95%) | Mean ± SD | CI (95%) |
| **Anti-CD26 IgM (µg/mL)** | 13.51 ± 5.62* | 7.11-24.53 | 30.30 ± 20.49* | 21.21-39.38 | 27.92 ± 18.08 | 23.16-32.67 | 16.85 ± 15.15* | 6.67-27.03 | 32.94 ± 21.82 | 20.85-45.02 |
| **Anti-CD26 IgA (µg/mL)** | 1.28 ± 0.27 ± | 0.85-1.87 | 1.76 ± 0.92* | 1.35-2.17 | 2.32 ± 1.51* | 1.92-2.72 | 1.81 ± 1.44* | 0.85-2.78 | 1.34 ± 0.71* | 0.95-1.73 |
| **Anti-CD26 IgG (µg/mL)** | 2.14 ± 0.64 | 0.98-3.58 | 5.60 ± 3.13 | 4.21-6.98 | 6.08 ± 3.15 | 5.25-6.91 | 5.69 ± 3.68 | 3.21-8.16 | 5.62 ± 2.83 | 4.05-7.19 |
| **Total IgM (mg/mL)** | 2.37 ± 124* | 0.73-3.72 | 3.26 ± 3.28 ± | 1.80-4.71 | 5.22 ± 3.95* | 4.18-6.26 | 2.23 ± 2.26* | 0.71-3.74 | 4.02 ± 3.92 | 1.85-6.19 |
| **Total IgA (mg/mL)** | 2.28 ± 0.27 | 1.91-2.60 | 5.56 ± 2.04 | 4.65-6.46 | 5.86 ± 1.82* | 5.38-6.34 | 5.46 ± 1.11 | 4.71-6.21 | 4.81 ± 1.38* | 4.05-5.58 |
| **Total IgG (mg/mL)** | 12.28 ± 2.79 | 8.27-16.60 | 17.73 ± 12.68 | 12.11-23.35 | 24.23 ± 17.1* | 19.74-28.73 | 15.73 ± 11.35* | 8.10-23.35 | 22.85 ± 12.76 | 15.78-29.91 |
| **Ratio IgM (µg/mg)** | 7.12 ± 5.19 | | 20.75 ± 25.75 | | 10.42 ± 13.12* | | 26.36 ± 33.05* | | 30.79 ± 51.75* | |
| **Ratio IgA (µg/mg)** | 0.57 ± 0.15 | | 0.49 ± 0.67 | | 0.47 ± 0.63* | | 0.32 ± 0.24* | | 0.29 ± 0.16* | |
| **Ratio IgG (µg/mg)** | 0.19 ± 0.07 | | 0.50 ± 0.78 | | 0.96 ± 1.82* | | 0.79 ± 1.05 | | 0.29 ± 0.17* | |

| | | | | | | | | | | |
|---|---|---|---|---|---|---|---|---|---|---|
| n= number of samples; BT= biological therapy; CI= confidence interval; SD= standard deviation. * Values significantly different among groups (not specified) at p < 0.05 with Student's ttest + Values significantly different to those of no biological therapy (No BT) group at p < 0.05 with Dunnett's t test. | | | | | | | | | | |

**Table 3. Ratios of total and anti-CD26 Ig isotypes in control and RA patients**

| | Controls (n=25) | RA patients (n=106) |
|---|---|---|
| | Mean ± SD | Mean ± SD |
| Total Ig ratio A/M | 1.3 ± 1.0 | 3.7 ± 6.7** |
| Total Ig ratio G/A | 5.4 ± 1.3 | 4.8 ± 7.3 |
| Total Ig ratio G/M | 6.7 ± 5.2 | 11.3 ± 18.6* |
| Auto (anti-CD26) Ig ratio A/M | 0.11 ± 0.04 | 0.13 ± 0.21 |
| Auto (anti-CD26) Ig ratio G/A | 1.7 ± 0.6 | 7.5 ± 27.1 |
| Auto (anti-CD26) Ig ratio G/M | 0.17 ± 0.06 | 0.32 ± 0.35** |

| | | |
|---|---|---|
| n= number of samples; SD= standard deviation. * Values significantly different among groups (not specified) at p < 0.05 with Student's t test. ** Values significantly different among groups (not specified) at p < 0.001 with Student's t test | | |

### Example 4.- Diagnostic value of anti-CD26/DPP-IV antibody levels

As our cohort of RA patients showed elevated titers of anti-CD26 autoantibodies, pointing to their utility to follow patients' responsiveness to therapy at individual level, we tested whether any of the isotype titers could have diagnostic value.

### 4.1 Anti-CD26/DPP-IV Ig levels in the whole cohort

The patients of the cohort under study have already been diagnosed and are undergoing therapy. Thus, this group of subjects is not ideal for the assessment of the diagnostic value of anti-CD26 antibody titers. However, the IgG isotype titers have shown to remain unchanged with the undergoing therapies and may thus be a good indicator.

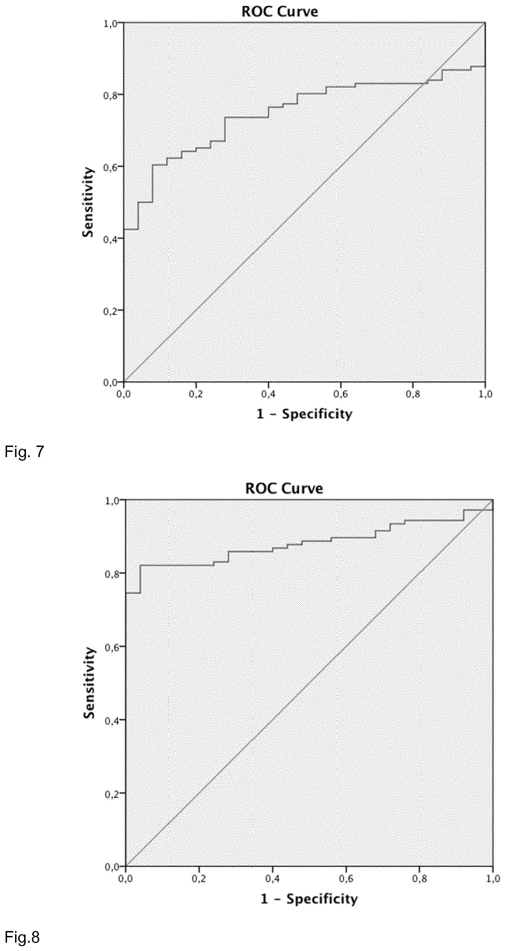

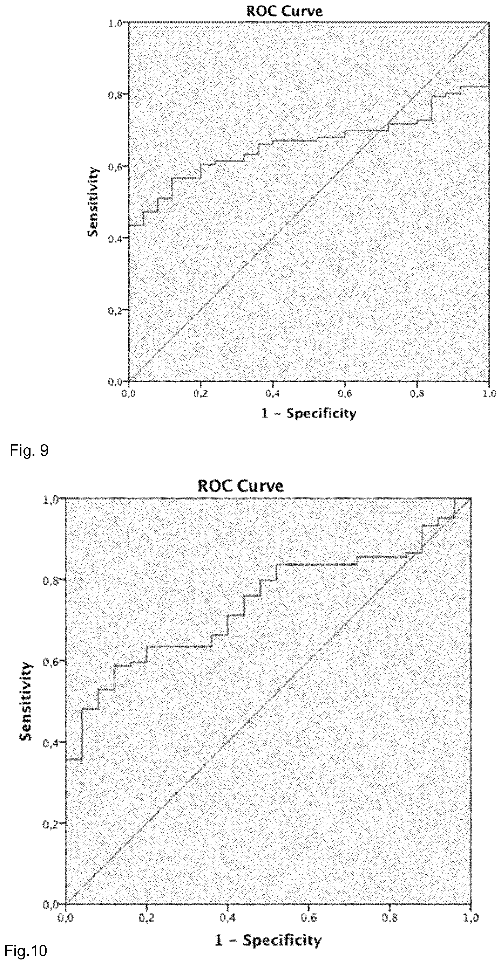

In addition, to assay the performance of the biomarkers in a screening/diagnostic test, ROC curves have been prepared representing the obtained sensitivity vs 1-specificity values for the anti-CD26 of the IgG, IgM and IgA isotypes, finding the following AUCs and Specificity (E) and Sensibility (S) values for the calculated optimal cut-off:
- anti-CD26 IgM (Fig.7): AUC (IC 95%): 0.748 (0.666-0.830); cut-off 19 microg/ mL, 62% S, 88% E
- anti-CD26 IgG (Fig.8): AUC (IC 95%): 0.881 (0.825-0.938); cut-off 2.85 microg/ mL, 82% S, 96% E
- anti-CD26 IgA (Fig.9): AUC (IC 95%): 0.661(0.573-0.749); cut-off 1.5 microg/ mL, 60% S, 80% E
- anti-CD26 IgG/total IgG (Fig.10): AUC(IC 95%): 0.741 (0.653-0.829); cut-off 0.24 microg/ mg, 60% S, 84% E

These cut-offs are, respectively, 1.41, 1.33, 1.17 and 1.26 -fold than the reference values (mean of healthy donors' biomarker concentrations).

### 4.2 Anti-CD26/DPP-IV antibody levels in patients undergoing DMARDs but no biological therapy

We then tested the group undergoing DMARDs but no biological therapy (noBT), which would better resemble undiagnosed patients and/or patients closer to pre-RA in the sense that these therapies do not change CD26 expression at least in the leukocyte populations (Ellingsen T et al. Scand J Immunol. 2007. 66: 451-7). A sensitivity of 77% (17/22) is found for the anti-CD26 IgG isotype (with a cut-off 2.85 microg/mL). A similar result, 73% (16/22) sensitivity is found for the anti-CD26 IgM (with a cut-off 20 microg/mL) and IgA 64% (with a cut-off 1.7 microg/mL) titers (Figure 1). To note, only 3 out of 22 patients were negative for both IgG and IgM isotypes and 2/22 for the three.

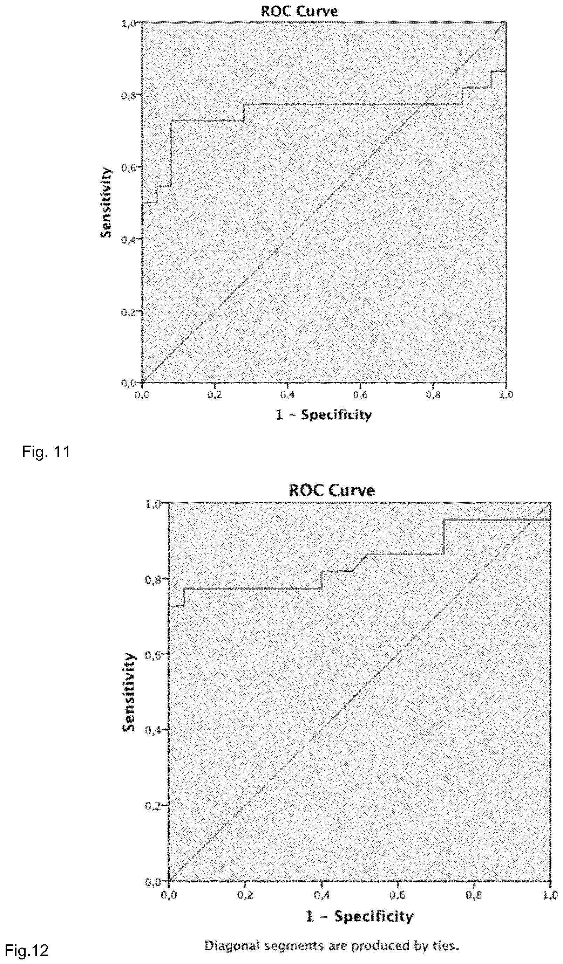

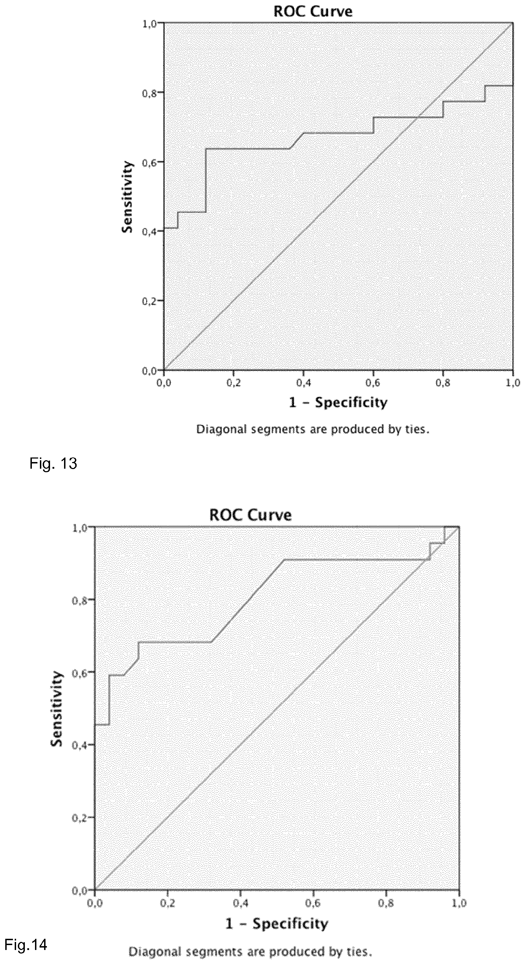

In addition, to assay the performance of the biomarkers in a screening/diagnostic test, ROC curves have been prepared representing the obtained sensitivity vs 1-specificity values for the anti-CD26 of the IgG, IgM and IgA isotypes, finding the following AUCs and Specificity (E) and Sensibility (S) values for the calculated optimal cut-off:
- anti-CD26 IgM (Fig.11): AUC (IC 95%): 0.751 (0.583-0.919); cut-off 20 microg/mL, 73% S, 92% E
- anti-CD26 IgG (Fig.12): AUC (IC 95%): 0.846 (0.718-0.975); cut-off 2.85 microg/mL, 77% S, 96% E
- anti-CD26 IgA (Fig.13): AUC (IC 95%): 0.672 (0.495-0.848); cut-off 1.7 microg/mL, 64% S, 80% E
- anti-CD26 IgG/total IgG (Fig.14): AUC (IC 95%): 0.804 (0.670 -0.938); cut-off: 0.24 microg/mg (same as whole cohort), 68%S, 84%E; and with cut-off: 0.25 microg/mL, 68%S, 88%E

These cut-offs are, respectively, 1.48, 1.33, 1.33 and 1.26 -fold than the reference values (mean of healthy donors' biomarker concentrations).

It is noted that an important difference between the inventors' results and those previously published by Cuchacovich et al. (Cuchacovich M et al. Clin Exp Rheumatol. 2001. 19: 673-80 is that in the previous study higher anti-CD26 antibody titers were found in RA and other autoimmune diseases, in particular of the IgA isotype. However, the inventors have found elevated levels of the three classes tested but particularly for the IgM and IgG isotypes, which in fact showed better diagnostic values compared to the IgA isotype. It should be pointed that Cuchacovich et al. only discloses ratios of auto-Abs/total Ig for each isotype whereas under Example 3 above are presented the results for each of these individual determinations and for the ratio, and indeed show that anti-CD26 Ig levels of each isotype were independent to changes in the total levels of each isotype. The differences for the anti-CD26 antibody titers between both studies may also be related in part to the fact that we have used as an antigen for the ELISA a recombinant sCD26 protein instead of sCD26 antigen isolated from patient sera, which has been reported to be a more complex molecule (Cuchacovich M et al. Clin Exp Rheumatol. 2001. 19: 673-80, Lambeir AM et al. Crit Rev Clin Lab Sci. 2003. 40: 209-94, Cordero OJ et al. Cancer Immunol Immunother. 2009. 58(11):1723-47).

### Example 5.- Diagnostic value of total IgA antibody levels

Interestingly, when performing ROC curves for anti-CD26 antibodies, total antibodies and the ratios thereof for the IgA, IgM and IgG isotypes, the inventors have observed that total IgA antibodies have shown to be a very good discriminator between healthy donors (HD) and the RA patient population both for the whole cohort (see Figure 15, AUC (IC 95%) of 0.979 (0.953-1.000); cut-off: 0.24 microg/mL, 60% S, 84% E) and the no biological therapy group of patients, which is to be assimilated to undiagnosed or pre-RA (see Figure 16, AUC (IC 95%) of 0.947(0.859 -1.000), for cut-off: 2.5 mg/mL, 96%S, 76%E; and for cut-off: 3 mg/mL, 91%S, 100%E).

### Example 6.- Correlations of anti-CD26/DPP-IV antibody levels with disease activity parameters of RA patients

The inventors have previously shown correlations between the DAS28 score, the DAS28 components (clinical: TJC, SJC, PGA, and laboratory variables: ESR and CRP, or other RA activity markers (HAQ, haemoglobin, platelet and haematocrit) with T cell subsets defined by the CD26 surface expression (Cordero OJ et al. PLoS One. 2015. 10(7):e0131992).

### 6.1 Anti-CD26/DPP-IV antibody levels in the whole cohort

Here, disease activity correlations were analysed first with total levels of each isotype, which include rheumatoid factor (RF) and ACPA, in the whole cohort. Total titers of the three Ig classes do not correlate with the DAS28, however some statistically significant correlations are still found between total titers for the three isotypes and ESR (r= 0.272, p=0.005 for IgM; r= 0.213, p=0.028 for IgG; r= 0.280, p=0.004 for IgA). Moreover, IgG negatively correlate with TJC and total IgM with HAQ.

Anti-CD26 levels do neither correlate with the DAS28 score in the whole cohort. However, the three anti-CD26 auto-Ab Ig titers correlate negatively with TJC (r= -0.272, p=0.036 for IgM; r= - 0.210, p=0.032 for IgG; r= -0.273, p=0.005 for IgA); in addition, the anti-CD26 IgG levels negatively correlate with the haematocrit and anti-CD26 IgA titers with ESR (r= -0.360, p<0.001 for the latter). Finally, the ratios of IgG and IgA isotypes correlated with the CRP.

### 6.2 Anti-CD26/DPP-IV antibody levels according to therapy

To check the effect that any of the different therapies may have on the correlations between the antibody levels and clinical scores, the same analysis (plus internal controls) was performed for each group of patients undergoing different therapies. In the no BT group, whereas strong correlations between all total Ig isotype titers are seen (and they show correlations with the platelet count) only anti-CD26 IgA and IgM isotype titers do show a correlation between them and only the IgA isotype ratio show a very strong correlation with TJC (r= 0.704, p<0.001).

In the anti-TNF group, total IgA titers do not correlate with titers of the other two isotypes, showing only a positive correlation with the ESR, positively (data not shown). Total IgM titers also correlate positively with Hb and haematocrit and negatively with the CRP whereas IgG titers correlate negatively with the ESR and the platelet count (data not shown). Very interestingly, anti-CD26 IgA titers strongly correlate with TJC and DAS28 (Figure 2A and 2B) and the anti-CD26 IgM levels positively correlate with TJC (r= 0.295, p=0.024). No correlations could be found for the IgG isotype.

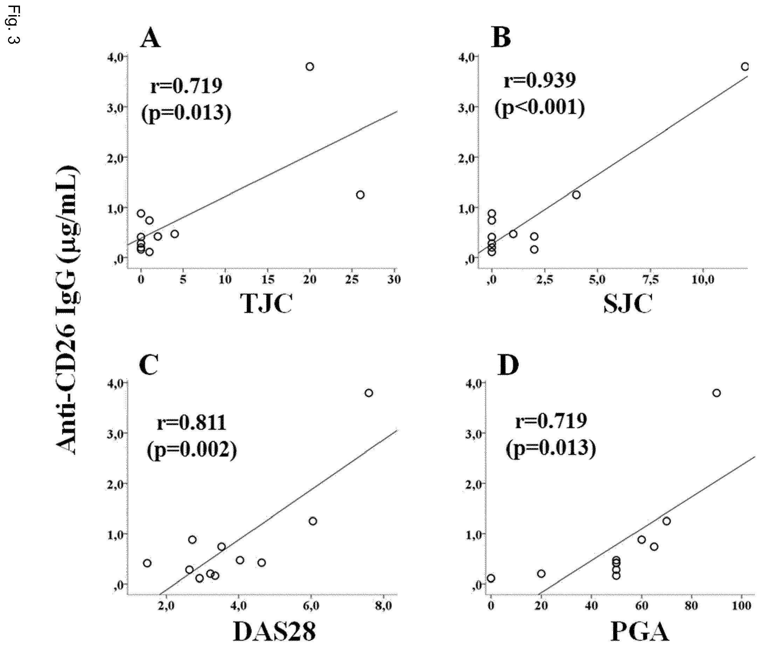

However, in the anti-CD20 group, these anti-CD26 IgG titers correlate very strongly with TJC, SJC, DAS28 and PGA (Figure 3A-D). No correlations were found for the other two isotypes, although anti-CD26 IgG and IgA titers correlated between them. In this group, total isotype titers do not correlate among them and the only correlations detected were the IgM isotype ratio with both HAQ and PGA and the IgG isotype ratio with platelets (data nor shown).

Finally, in the anti-IL6R/Ig-CTLA4 group, whereas total IgA levels do not correlate with the other isotype titers (as in the anti-TNF group) and anti-CD26 IgA and IgM titers do not correlate between them (on the contrary to the noBT group), auto-Abs titers in the other combinations (IgA with IgG and IgM with IgG) do it. In this group, only the IgG isotype ratio correlates with TJC and total IgG with the hematocrit.

Altogether, the present data suggest: a) a specific relation between anti-CD26 auto-antibodies with the joints and disease activity, b) these antibody levels are differently affected by each therapy, and c) they provide a different information compared to the most frequently disease activity parameters used at present (ESR, CRP, platelet count, Hb levels or haematocrit).

### Example 7.- Higher anti-CD26 antibody titers are detected in smokers

Tobacco is an environmental factor triggering autoimmunity in ACPA-positive RA (Reynisdottir G et al. Arthritis Rheum. 2014. 66(1):31-9. doi: 10.1002/art.38201, Catrina Al et al. Nat Rev Rheumatol. 2014. 10(11):645-53. doi: 10.1038/nrrheum.2014.115, Vossenaar ER et al. Arthritis Res Ther. 2004. 6: 107-111). We decided to compare mean titers for the three anti-CD26 isotypes dividing the whole RA patient cohort in three groups according to their smoking status, current (n=18), past (n=28) or non-smokers (n=59). Whereas IgA titers were similar among the three groups, both IgM and IgG showed higher levels in past smokers in particular (26.0 µg/mL in non-smokers compared to 31.8 µg/mL in past smokers for IgM, and 5.5 µg/mL to 6.7 µg/mL for IgG), although these differences did not achieve statistical significance.

We then analysed the RA cohort taking into account therapy (Table 4). Interestingly, in the no-BT group, anti-CD26 IgM titers are significantly higher in past smoker patients compared to non-smokers. Although there are few current smoker patients in this group, they also showed higher auto-antibody titers. The anti-CD26 IgG isotype also showed a trend to higher titers in past smokers compared to the other groups, although this trend did not achieve statistical significance. To note, whereas anti-CD26 IgG or IgA levels do not change among the different therapies, the anti-CD26 IgM levels were attenuated in past smokers in the anti-TNF group and in all patients in the anti-CD20 treatment group; however, in the anti-IL6R/Ig-CTLA4 treatment group non-smokers showed increased levels for the CD26 IgM isotype (Table 4).

**Table 4. Titers of anti-CD26 Ig isotypes in RA patients under different therapies according to their smoking status**

| | | **No BT (n=22)** | | **Anti-TNFα BT (n=58)** | | **Anti-CD20 BT (n=11)** | | **Anti-IL6R/Ig-CTLA4 BT (n=15)** | |
|---|---|---|---|---|---|---|---|---|---|
| | | Mean ± SD (n) | | Mean ± SD (n) | | Mean ± SD (n) | | Mean ± SD (n) | |
| **Anti-CD26 IgM (µg/ml)** | Non smokers | 22.4 ± 18.0 | (12) | 25.4 ± 19.0 | (33) | 13.5 ± 7.7 | (3) | 37.1 ± 25.5 | (10) |
| | Smokers | 28.6 ± 3.8 | (2) | 33.0 ± 16.5 | (13) | 9.7 ± 9.1 | (4) | - | - |
| | Past smokers | 42.6 ± 21.7* | (8) | 29.6 ± 18.0 | (11) | 25.6 ± 22.0 | (4) | 24.6 ± 8.3 | (5) |
| **Anti-CD26 IgG (µg/mL)** | Non smokers | 4.6 ± 3.2 | | 5.9 ± 3.4 | | 4.8 ± 4.0 | | 5.7 ± 3.1 | |
| | Smokers | 8.1 ± 1.6 | | 6.0 ± 2.9 | | 4.0 ± 2.0 | | - | - |
| | Past smokers | 6.4 ± 2.9 | | 6.9 ± 2.7 | | 7.9 ± 4.0 | | 5.6 ± 2.7 | |
| **Anti-CD26 IgA (µg/mL)** | Non smokers | 1.6 ± 0.8 | | 2.4 ± 1.6 | | 1.4 ± 2.1 | | 1.3 ± 0.8 | |
| | Smokers | 1.7 ± 1.4 | | 2.1 ± 1.3 | | 2.1 ± 0.8 | | - | - |
| | Past smokers | 2.0 ± 1.0 | | 2.4 ± 1.6 | | 2.1 ± 1.2 | | 1.5 ± 0.7 | |

| | | | | | | | | | |
|---|---|---|---|---|---|---|---|---|---|
| n= number of samples; BT= biological therapy; SD= standard deviation, * Value significantly different at p < 0.05 with Student's t test between groups classified according to their smoking status (past smokers vs non smokers). Groups were compared only inside each type of therapy. | | | | | | | | | |

### Example 8.- ACPA Test

The obtained results, combining the well-established second-generation Elia™ CCP tool in the clinical practice together with our in-house ELISA for anti-CD26 auto-Abs (only the IgG isotype was tested), enable to conclude that auto anti-CD26 Abs are not ACPA. When supernatants of serum from both healthy donors and RA patients were recovered from the ACPA test and tested for the presence of anti-CD26 autoantibodies, an absorbance similar to that obtained with the same serum sample tested only using our anti-CD26 ELISA test (not shown) could be detected (Figure 4A, RA patients showing highest absorbance data). The ACPA test showed the results expected, with higher levels in the serum of the three patients analyzed compared to three control samples from healthy donors, except for one of these, which was also positive (a candidate for developing arthritis) (Figure 4B, dark gray).

To verify these results, we also performed the inverse analysis, where supernatants recovered from the anti-CD26 ELISA were used later in the ACPA test. Although absorbances were similar to those obtained after the direct incubation of the same serum in the ACPA test, there were minor differences in four of the samples and more significant in two of them, one healthy control and one RA patient (Figure 4B, in clear gray). In this case, when proteins captured in the assay were eluted from the plate well, a minor positive signal was observed (data not shown), pointing to the possibility that a minor part of ACPA autoantibodies may also bind to CD26.

### Example 9.- Serum anti-CD26 auto-antibodies and their correlations with DPP-IV activity and sCD26 concentrations in the cohort of RA patients under different therapies

### 9.1 Serum anti-CD26 auto-antibodies and their correlations with DPP-IV activity and sCD26 concentrations in the whole cohort

As determined for the healthy control group (see Example 2 above), we analysed the correlations of the Ig isotypes and their ratios with the serum DPP-IV activity and sCD26 concentrations in the whole cohort of patients. Near statistically significant positive correlations were found between total IgG and anti-CD26 IgG with the sCD26 concentration but not enzymatic activity for the whole cohort.

### 9.2 Serum anti-CD26 auto-antibodies and their correlations with DPP-IV activity and sCD26 concentrations according to therapy

When the same analysis was performed for each of the patients groups according to therapy many interesting correlations were found. In the group without BT (noBT), total levels of IgA and IgM positively correlate with the DPP-IV activity but not with the sCD26 concentration (Figure 5A) whereas in the anti-IL6R/Ig-CTLA4 group, total IgM and IgG titers negatively correlate with the DPP-IV activity (Figure 5D). In the anti-TNFa group, which present increased DPP-IV activity levels (Cuchacovich M et al. Clin Exp Rheumatol. 2001. 19: 673-80), in addition to the total IgA and IgM titers, the anti-CD26 IgG titers were also positively correlated with DPP-IV activity (Figure 5B). Only in this group (Figure 5B) total titers of IgM and IgG and anti-CD26 IgG and IgM titers correlate positively with the sCD26 concentration, whereas in the antiCD20 group, showing decreased levels of IgM autoantibodies, both anti-CD26 IgM titers and ratio show a strong negative correlation with the sCD26 concentration (Figure 5C).

These results point to the idea that: a) auto-Ab levels do not relate to the reduction of their Ag (sCD26) concentrations in RA patients, since the only correlation seen was for the anti-TNF group, but a positive correlation instead; b) DPP-IV activity and sCD26 concentration, reduced in RA patients and affected by therapies (Cordero OJ et al. PLoS One. 2015. 10(7):e0131992), are related to the same pathway that enhances anti-CD26 autoAb titers; c) together with the fact that different levels of correlation between DPP-IV activity and sCD26 concentration are found in each group of patients (they do not correlate in the no-BT and in the anti-IL6R/Ig-CTLA4 group and correlate strongest in the anti-CD20 group, data not shown), we also conclude that DPP-IV activity and sCD26 concentration are implicated in RA pathogenesis in different ways; and d) finally, different therapies have a profound impact in these pathways even in opposite ways.

Interestingly Cuchacovich et al showed a negative correlation between the (circulating levels of DPP-IV and titers of anti-CD26 IgA autoantibodies in RA (Cuchacovich M et al. Clin Exp Rheumatol. 2001. 19: 673-80, Cuchacovich M et al. Clin Diagn Lab Immunol. 2002. 9:1253-9), a correlation which was not found in the present study. On the contrary, when correlations were found these were positive and mostly for the anti-CD26 IgG and IgM isotypes. We did found a negative correlation in the anti-CD20 group of patients but in this group therapy was found to decrease the autoantibody levels in general. It can be concluded, therefore, that impairment of DPP-IV activity and sCD26 concentration in RA patients is related to the same pathway that enhances anti-CD26 auto-Ab titers, which thus do not appear to be involved in the clearance of sCD26 from serum as the more prominent role.

### Example 10.- Serum anti-CD26 auto-antibodies and their relationship with ACPA analysis.

Early diagnosis and treatment of RA has been associated with positive treatment outcome and RA remission. However, the sensitivity of anti-citrullinated peptides antibodies (ACPA), the most specific biomarker for RA up to date, is about 75% in established RA compared with 55% in early RA and 40% in very early RA (Nicaise R P, et al., Arthritis Res Ther 2008;10:R142; Ohmura K, et al. Rheumatology 2010;49:2298-304).

The serum anti-CD26 levels in a cohort of RA patients under different biological and non-biological therapies were measured as disclosed in Example 2 (see Table 3). With the cut-off points obtained for serum IgA, IgM and IgG anti-CD26 titers using also a healthy donor group (n=25), we had obtained high sensitivity and specificity data (see Example 4). Here, we compared the positivity for both anti-CD26 and ACPA auto-antibodies.

ACPA levels in the patients' serum were measured using a standard EliA™ CCP - Fully Automated Anti-CCP second generation detection test (Phadia, Thermo Fisher Scientific). In this automated enzyme-linked immunosorbent assay (ELISA), anti-CCP antibodies of the isotype IgG were detected in the samples and the result calibrated against a standarized curve. Samples with a value of 10 EliA U/mL were considered ACPA positive, in accordance with the manufacturer's instructions. Positivity with respect to anti-CD26 antibodies was assigned to values above the cut-off levels defined in Example 4.

Testing anti-CCP showed that 22 diagnosed RA patients were ACPA-negative. In this group of patients sensitivity of 77% (17/22 positives) was found for the anti-CD26 IgG isotype, which is the same we found with the same cut-off in the group of patients under DMARDs (which may be closer to undiagnosed people). A similar result, 14/22 instead of 16/22 positives, was found for the anti-CD26 IgA. However, the results were worse for the IgM (10/22 instead of 16/22 positives). Interestingly, 100% of ACPA-negative patients were total IgA positives. The obtained results suggest that auto-antibodies against CD26/DPP-IV participate in RA pathogenesis in an independent way than ACPA, and may serve for earlier diagnosis.

**Table 5. Frequencies of positivity for anti-CD26 Ig isotypes in RA patients classified according to their ACPA status**

| | **ACPA** - **(n=22)** | **ACPA + (n=53)** | **Total (n=106)** |
|---|---|---|---|
| | (%) | (%) | (%) |
| **Anti-CD26 IgM** | 45 | 66 | 62 |
| **Anti-CD26 IgA** | 64 | 60 | 60 |
| **Anti-CD26 IgG** | 77 | 87 | 82 |

## Claims

1. *In vitro* method for the screening of an inflammatory rheumatic disease in a subject comprising the following steps:
a) determining the levels of anti-CD26 IgG antibodies in an antibody-containing sample isolated from said subject;
b) comparing the levels in said antibody-containing sample with a reference value;
wherein an increase of the anti-CD26 IgG antibody levels in the subject sample with regard to said reference value is indicative of inflammatory rheumatic disease.

2. *In vitro* method for the diagnosis of an inflammatory rheumatic disease in a subject, said method comprising the steps a) and b) of claim 1,
wherein an increase of the anti-CD26 IgG antibody levels in the subject sample with regard to said reference value is indicative of inflammatory rheumatic disease.

3. *In vitro* method for classifying subjects as i) healthy subjects or as ii) subjects having an inflammatory rheumatic disease, said method comprising the steps a) and b) of claim 1, wherein an increase of the anti-CD26 IgG antibody levels in the subject sample with regard to said reference value is indicative of inflammatory rheumatic disease.

4. The method according to any of claims 1 to 3, wherein said subject has not previously received or is not undergoing any biological treatment against said inflammatory rheumatic disease, preferably wherein said biological treatment is selected from the group consisting of:
a. Anti-TNF alpha agents;
b. Anti-CD20 agents; and
c. Anti-IL6R agents and/or CTLA4-Fc fusion proteins.

5. The method according to any of claims 1 to 3, wherein said subject has not previously received or is not undergoing any treatment against said inflammatory rheumatic disease.

6. *In vitro* method of disease monitoring and/or monitoring responsiveness to a treatment in a subject having an inflammatory rheumatic disease, said method comprising the steps
a) and b) of claim 1,
wherein an increase of the anti-CD26 IgG antibody levels in the subject sample with regard to said reference value is indicative of inflammatory rheumatic disease.

7. *In vitro* method for the classification of a subject as responder to a treatment to an inflammatory rheumatic disease,
said method comprising the steps a) and b) of claim 1,
wherein an increase of the anti-CD26 IgG antibody levels in the subject sample with regard to said reference value is indicative of lack of response.

8. The method according to any of claims 1 to 7, wherein said reference value corresponds to the anti-CD26 IgG antibody mean levels in healthy subjects.

9. The method according to any of claims 1 to 8, wherein said inflammatory rheumatic disease is selected from the group consisting of rheumatoid arthritis, lupus erythematosus, Sjogren syndrome, juvenile rheumatoid arthritis, psoriatic arthritis and spondyloarthropathy, preferably wherein said inflammatory rheumatic disease is rheumatoid arthritis.

10. The method according to any of claims 1 to 9, said method comprising the steps a) and b) of claim 1, further comprises:
a) determining the anti-CD26 antibody levels of the IgM and/or IgA isotype in said antibody-containing sample;
b) comparing the anti-CD26 IgM and/or IgA antibody levels in said antibody-containing sample with a reference value;
wherein an increase of the anti-CD26 IgG antibody levels value and of the anti-CD26 IgM and/or IgA antibody levels in the subject sample with regard to the corresponding reference value is indicative of disease;
wherein the respective reference value corresponds to the mean levels in healthy subjects.

11. The method according to any of claims 1 to 10, wherein said subject is a human subject.

12. The method according to any of claims 1 to 11, wherein said antibody-containing sample is whole blood, serum or plasma, preferably serum.

13. The method according to any of claims 1 to 12, wherein said method further comprises storing the method results in a data carrier, preferably wherein said data carrier is a computer readable medium.

14. A computer implemented method, wherein the method is as defined in any of claims 1 to 13.

15. Use of a kit for the screening, diagnosis, and/or monitoring of an inflammatory rheumatic disease; and/or for monitoring responsiveness to a treatment in an antibody-containing sample isolated from a subject, wherein said kit comprises:
- a reagent for determining the levels of anti-CD26 antibody of the IgG isotype; and
- optionally, instructions for the use of said reagent in determining the anti-CD26 antibody levels of the IgG isotype in an antibody-containing sample isolated from said subject.

## Patentansprüche

1. *In vitro*-Verfahren für die Untersuchung einer rheumatischen Entzündungskrankheit in einem Individuum, welches die folgenden Schritte umfasst:
a) das Bestimmen der Anti-CD26-IgG-Antikörperspiegel in einer Antikörper enthaltenden Probe, welche aus dem genannten Individuum isoliert wurde;
b) das Vergleichen der Spiegel in der genannten Antikörper enthaltenden Probe mit einem Referenzwert;
wobei eine Erhöhung der Anti-CD26-IgG-Antikörperspiegel in der Probe des Individuums in Bezug auf den genannten Referenzwert auf eine rheumatische Entzündungskrankheit schließen lässt.

2. *In vitro*-Verfahren für die Diagnose einer rheumatischen Entzündungskrankheit in einem Individuum, wobei das genannte Verfahren Schritte a) und b) aus Anspruch 1 umfasst, wobei eine Erhöhung der Anti-CD26-IgG-Antikörperspiegel in der Probe des Individuums in Bezug auf den genannten Referenzwert auf eine rheumatische Entzündungskrankheit schließen lässt.

3. *In vitro*-Verfahren für die Klassifizierung von Individuen als i) gesunden Individuen oder als ii) Individuen, welche eine rheumatische Entzündungskrankheit aufweisen, wobei das genannte Verfahren Schritte a) und b) aus Anspruch 1 umfasst, wobei eine Erhöhung der Anti-CD26-IgG-Antikörperspiegel in der Probe des Individuums in Bezug auf den genannten Referenzwert auf eine rheumatische Entzündungskrankheit schließen lässt.

4. Verfahren nach einem der Ansprüche 1 bis 3, wobei das genannte Individuum hat zuvor keine biologische Behandlung gegen die genannte rheumatische Entzündungskrankheit bekommen oder sich derselben derzeit nicht unterzieht, wobei vorzugsweise die genannte biologische Behandlung aus der Gruppe bestehend aus:
a. Anti-TNF-Alpha-Mitteln;
b. Anti-CD20-Mitteln; und
c. Anti-IL6R-Mitteln und/oder CTLA4-Fc-Fusionsproteinen,
ausgewählt wird.

5. Verfahren nach einem der Ansprüche 1 bis 3, wobei das genannte Individuum hat zuvor keine Behandlung gegen die genannte rheumatische Entzündungskrankheit bekommen oder sich derselben derzeit nicht unterzieht.

6. *In vitro*-Verfahren für die Krankheitsüberwachung und/oder für die Überwachung der Ansprechbarkeit auf eine Behandlung in einem Individuum, welches eine rheumatische Entzündungskrankheit aufweist, wobei das genannte Verfahren Schritte a) und b) aus Anspruch 1 umfasst, wobei eine Erhöhung der Anti-CD26-IgG-Antikörperspiegel in der Probe des Individuums in Bezug auf den genannten Referenzwert auf eine rheumatische Entzündungskrankheit schließen lässt.

7. *In vitro*-Verfahren für die Klassifizierung eines Individuums als ansprechendes Individuum auf eine Behandlung gegen eine rheumatische Entzündungskrankheit, wobei das genannte Verfahren Schritte a) und b) aus Anspruch 1 umfasst, wobei eine Erhöhung der Anti-CD26-IgG-Antikörperspiegel in der Probe des Individuums in Bezug auf den genannten Referenzwert auf ein fehlendes Ansprechen schließen lässt.

8. Verfahren nach einem der Ansprüche 1 bis 7, wobei der genannte Referenzwert den mittleren Anti-CD26-IgG-Antikörperspiegeln in gesunden Individuen entspricht.

9. Verfahren nach einem der Ansprüche 1 bis 8, wobei die genannte rheumatische Entzündungskrankheit aus der Gruppe bestehend aus rheumatoider Arthritis, Lupus erythematodes, Sjögren-Syndrom, juveniler rheumatoider Arthritis, Psoriasis-Arthritis und Spondyloarthropathie ausgewählt wird, wobei vorzugsweise die genannte rheumatische Entzündungskrankheit rheumatoide Arthritis ist.

10. Verfahren nach einem der Ansprüche 1 bis 9, wobei das genannte Verfahren Schritte a) und b) aus Anspruch 1 umfasst, zusätzlich umfassend:
a) das Bestimmen der Anti-CD26-Antikörperspiegel des IgM- und/oder IgA-Isotyps in der genannten Antikörper enthaltenden Probe;
b) das Vergleichen der Anti-CD26-IgM- und/oder -IgA-Antikörperspiegel in der genannten Antikörper enthaltenden Probe mit einem Referenzwert;
wobei eine Erhöhung des Wertes der Anti-CD26-IgG-Antikörperspiegel und der Anti-CD26-IgM- und/oder -IgA-Antikörperspiegel in der Probe des Individuums in Bezug auf den entsprechenden Referenzwert auf Krankheit schließen lässt; wobei der jeweilige Referenzwert den mittleren Spiegeln in gesunden Individuen entspricht.

11. Verfahren nach einem der Ansprüche 1 bis 10, wobei das genannte Individuum ein menschliches Individuum ist.

12. Verfahren nach einem der Ansprüche 1 bis 11, wobei die genannte Antikörper enthaltende Probe Vollblut, Serum oder Plasma, vorzugsweise Serum ist.

13. Verfahren nach einem der Ansprüche 1 bis 12, wobei das genannte Verfahren zusätzlich das Speichern der Verfahrensergebnisse in einem Datenträger umfasst, wobei vorzugsweise der genannte Datenträger ein computerlesbares Medium ist.

14. Computerimplementiertes Verfahren, wobei das Verfahren nach einem der Ansprüche 1 bis 13 ist.

15. Verwendung eines Kits für die Untersuchung, Diagnose und/oder Überwachung einer rheumatischen Entzündungskrankheit; und/oder für die Überwachung der Ansprechbarkeit auf eine Behandlung in einer Antikörper enthaltenden Probe, welche aus einem Individuum isoliert wurde, wobei das genannte Kit Folgendes umfasst:
- ein Reagens für die Bestimmung der Anti-CD26-Antikörperspiegel des IgG-Isotyps; und
- wahlweise, Anleitungen für die Verwendung des genannten Reagens bei der Bestimmung der Anti-CD26-Antikörperspiegel des IgG-Isotyps in einer Antikörper enthaltenden Probe, welche aus dem genannten Individuum isoliert wurde.

## Revendications

1. Procédé *in vitro* pour le dépistage d'une maladie rhumatismale inflammatoire chez un sujet comprenant les étapes suivantes :
a) déterminer les niveaux d'anticorps IgG anti-CD26 dans un échantillon contenant des anticorps isolé à partir dudit sujet ;
b) comparer les niveaux dans ledit échantillon contenant des anticorps avec une valeur de référence ;
dans lequel une augmentation des niveaux d'anticorps IgG anti-CD26 dans l'échantillon du sujet par rapport à ladite valeur de référence est indicative de maladie rhumatismale inflammatoire.

2. Procédé *in vitro* pour le diagnostic d'une maladie rhumatismale inflammatoire chez un sujet, ledit procédé comprenant les étapes a) et b) selon la revendication 1,
dans lequel une augmentation des niveaux d'anticorps IgG anti-CD26 dans l'échantillon du sujet par rapport à ladite valeur de référence est indicative de maladie rhumatismale inflammatoire.

3. Procédé *in vitro* pour le classement de sujets comme i) sujets en bonne santé ou comme ii) sujets ayant une maladie rhumatismale inflammatoire, ledit procédé comprenant les étapes a) et b) selon la revendication 1, dans lequel une augmentation des niveaux d'anticorps IgG anti-CD26 dans l'échantillon du sujet par rapport à ladite valeur de référence est indicative de maladie rhumatismale inflammatoire.

4. Procédé selon l'une quelconque des revendications 1 à 3, dans lequel ledit sujet n'a pas reçu au préalable ou n'est pas en train de recevoir un traitement biologique quelconque contre ladite maladie rhumatismale inflammatoire, de préférence dans lequel ledit traitement biologique est choisi parmi le groupe composé de :
a. agents anti-TNF alpha
b. agents anti-CD20 ; et
c. agents anti-IL6R et/ou protéines de fusion CTLA4-Fc.

5. Procédé selon l'une quelconque des revendications 1 à 3, dans lequel ledit sujet n'a pas reçu au préalable ou n'est pas en train de recevoir un traitement quelconque contre ladite maladie rhumatismale inflammatoire.

6. Procédé *in vitro* de surveillance de maladie et/ou de surveillance de réponse à un traitement chez un sujet ayant une maladie rhumatismale inflammatoire, ledit procédé comprenant les étapes a) et b) selon la revendication 1,
dans lequel une augmentation des niveaux d'anticorps IgG anti-CD26 dans l'échantillon du sujet par rapport à ladite valeur de référence est indicative de maladie rhumatismale inflammatoire.

7. Procédé *in vitro* pour le classement d'un sujet comme répondeur à un traitement d'une maladie rhumatismale inflammatoire,
ledit procédé comprenant les étapes a) et b) selon la revendication 1,
dans lequel une augmentation des niveaux d'anticorps IgG anti-CD26 dans l'échantillon du sujet par rapport à ladite valeur de référence est indicative d'absence de réponse.

8. Procédé selon l'une quelconque des revendications 1 à 7, dans lequel ladite valeur de référence correspond aux niveaux moyens d'anticorps IgG anti-CD26.

9. Procédé selon l'une quelconque des revendications 1 à 8, dans lequel ladite maladie rhumatismale inflammatoire est choisie parmi le groupe composé d'arthrite rhumatoïde, lupus érythémateux, syndrome de Sjögren arthrite rhumatoïde juvénile, arthrite psoritique et spondyloarthropathie, de préférence dans lequel ladite maladie rhumatismale inflammatoire est arthrite rhumatoïde.

10. Procédé selon l'une quelconque des revendications 1 à 9, ledit procédé comprenant les étapes a) et b) selon la revendication 1, comprend en outre :
a) déterminer les niveaux d'anticorps anti-CD26 de l'isotype IgM et/ou IgA dans ledit échantillon contenant des anticorps ;
b) comparer les niveaux d'anticorps IgM et/ou IgA anti-CD26 dans ledit échantillon contenant des anticorps avec une valeur de référence ;
dans lequel une augmentation de la valeur des niveaux d'anticorps IgG anti-CD26 et des niveaux d'anticorps IgM et/ou IgG anti-CD26 dans l'échantillon du sujet par rapport à la valeur de référence correspondante est indicative de maladie ;
dans lequel la valeur de référence respective correspond aux niveaux moyens chez des sujets en bonne santé.

11. Procédé selon l'une quelconque des revendications 1 à 10, dans lequel ledit sujet est un sujet humain.

12. Procédé selon l'une quelconque des revendications 1 à 11, dans lequel ledit échantillon contenant des anticorps est du sang entier, du sérum ou du plasma, de préférence du sérum.

13. Procédé selon l'une quelconque des revendications 1 à 12, dans lequel ledit procédé comprend en outre le stockage des résultats du procédé dans un support de données, de préférence dans lequel ledit support de données est un moyen lisible par ordinateur.

14. Procédé mis en œuvre par ordinateur, dans lequel le procédé est selon l'une quelconque des revendications 1 à 13.

15. Utilisation d'une trousse pour le dépistage, diagnostic et/ou la surveillance d'une maladie rhumatismale inflammatoire, et/ou pour la surveillance de la réponse à un traitement dans un échantillon contenant des anticorps isolé à partir d'un sujet, dans laquelle ladite trousse comprend :
- un réactif pour déterminer les niveaux d'anticorps anti-CD26 de l'isotype IgG ; et
- optionnellement, des instructions pour l'utilisation dudit réactif dans la détermination des niveaux d'anticorps anti-CD26 de l'isotype IgG dans un échantillon contenant des anticorps isolé à partir dudit sujet.
